# EUROPEAN PATENT SPECIFICATION

(11) **EP 0 754 469 B1**
(45) Date of publication and mention of the grant of the patent: **13.03.2002**
(21) Application number: 96110090.6
(22) Date of filing: 21.06.1996
(51) Int. Cl.: A61M 5/32, A61M 5/50, A61M 39/06, A61M 25/06, A61B 5/15

(54) **Hypodermic needle assembly**
Nadelanordnung für Subkutaninjektionen
Ensemble aiguille hypodermique

(30) Priority: 23.06.1995 US 494283; 16.01.1996 US 587030; 19.03.1996 US 618624
(43) Date of publication of application: 22.01.1997
(73) Proprietor: Mahurkar, Sakharam Dhundiraj, Chicago Illinois 60660 (US)
(72) Inventor: Mahurkar, Sakharam Dhundiraj, Chicago Illinois 60660 (US)
(74) Representative: Grünecker, Kinkeldey, Stockmair & Schwanhäusser Anwaltssozietät

(56) References cited:
- DE-A- 3 833 138
- US-A- 5 338 311

## Description

### Field Of The Invention

The present invention generally relates to hypodermic needle syringe devices and, in particular, to such a needle-syringe assemblies which conceals the sharp point of the hypodermic needle following use.

### Background Of The Invention

A hypodermic needle has many applications in modern medicine. One application is to fit the hypodermic needle onto a syringe and to then insert the needle into a person's body for intra-muscular, subcutaneous, or intravenous injection of medications. A hypodermic needle entering into a patient's body is invariably contaminated by the patient's blood and body fluids. Following use of the needle, the needle presents a risk to physicians, nurses, and other health care personnel because the needle might transmit an infection or disease to such personnel if it were to accidentally puncture them. Thus, health care personnel are in constant danger of contracting infections and diseases, some of which may be deadly. Other potential victims of accidental needle punctures include sanitation workers which later dispose of garbage containing the hypodermic needle. The diseases which may be transmitted by a contaminated hypodermic needle include Immune Deficiency Virus, Hepatitis, Rabies, Kure, Encephalitis, and Arbor viruses. The outcome of contracting one of these diseases is often fatal because there are no known cures for any of these diseases. Often a needle puncture in a person's skin is so trivial that it remains unrecognized until the person becomes seriously ill.

The problem of suffering accidental needle punctures is well recognized. As a result, enormous inventive effort has been devoted to concealing the sharp needle point of hypodermic needles.

The deep-seated central veins, such as subclavian, jugular and femoral veins as well as arteries can only be accessed by negotiating a guidewire, through the puncturing needle, after disconnecting the syringe. The blood gushing out from the needle interferes with the visibility and insertion of the guidewire inside the needle. The blood also contaminates the area of operation and makes the blind insertion procedure even more difficult. The risks of needle stick are further multiplied by free sharp objects, scattered in a limited field. The present

invention also permits the insertion of the guidewire through the plunger itself without removing the syringe from the needle, as well as making the procedure essentially bloodless. It also avoids the needle puncture by retracting the needle before it comes out of the patient's body.

US-A-5 338 311 shows a needle-syringe assembly which is operable in a normal mode and convertible to a retraction mold. A barrel forms a hollow nozzle located at the distal end of the barrel. A plunger forms a coaxial cavity extending therethrough, and the plunger includes a helical slot exposing a proximal portion of the coaxial cavity. The barrel having a slot for engaging a lateral arm of the needle holder which extends therethrough through the outside. To switch from the normal mode to the retraction mode, the taper lock is disengaged by a rotary movement of the plunger relative to the barrel. By preventing rotary movement of the needle holder relative to the barrel, continued rotary movement of the plunger causes a lateral arm of the needle holder to ascend through the helical slot so that the needle holder retracts into the coaxial cavity of the plunger and the needle is concealed.

### Summary of the Invention

One object of the present invention is to provide an improved needle-syringe assembly which improves the acceptability of the assembly and which facilitates the operation of the assembly.

In accordance with the present invention, the foregoing objectives are realized by providing a needle-syringe assembly according to claim 1.

### Brief Description of the Drawings

FIG. 1 is an exploded plan view of a needle-syringe assembly embodying the present invention;
FIG. 2 is an enlarged longitudinal section of the needle-syringe assembly in FIG. 1 with the needle holder in the advanced position;
FIG. 3 is an enlarged fragmentary longitudinal section through a distal portion of the needle holder of the needle-syringe assembly in FIG. 1;
FIG. 4 is an enlarged view of the needle and needle holder assembly shown in FIG. 1;
FIG 5 is an enlarged fragmentary longitudinal section through a distal portion of the needle-syringe assembly in FIG. 2;
FIG. 6 is a section taken generally along line 6-6 in FIG. 2;
FIG. 7 is a section taken generally along line 7.7 in FIG. 6;
FIG. 8 is an enlarged exploded perspective view of the proximal end portion of the barrel of the needle-syringe assembly of FIGS. 1-7;
FIG. 9a is an end elevation of the barrel shown in FIG. 8;
FIGS. 10 and 11 are longitudinal sections of the needle-syringe assembly of FIGS. 1-9 showing the available range of axial movement of the plunger relative to the barrel; and
FIG. 12 is a longitudinal section of the needle-syringe assembly of FIGS 1-11 with the needle holder in the retracted position and the needle concealed by the barrel.
FIG. 13 is a longitudinal section similar to FIG. 5 but showing a modified construction of the barrel nozzle;
FIG. 14 is an enlarged side elevation, partially in section, of a modified needle holder for use with the barrel nozzle shown in FIG. 13;
FIG. 15 is a side elevation of the proximal end portion of a modified needle-syringe assembly embodying the invention;
FIG. 16 is a side elevation of the proximal end portion of a barrel for use with the plunger of FIG. 15;
FIG. 17 is an end elevation of the assembly shown in FIG. 16;
FIG. 18 is an exploded side elevation of the proximal end portion of a modified barrel construction:
FIG. 19 is an enlarged section taken along line 19-19 in FIG. 1;
FIG. 20 is a side elevation of the proximal end portion of a modified barrel design;
FIG. 21 is a section taken along line 21-21 in FIG. 20;
FIG. 22 is a side elevation, partially in section, of a needle-syringe assembly embodying the present invention;
FIG. 23 is a section taken generally along line 23-23 of FIG. 22;
FIG. 24 is a section taken generally along line 24-24 in FIG. 23, with the plunger removed;
FIG. 25 is a plan view of the latching element included in the needle-syringe assembly of FIG. 20;
FIG. 26 is the same view shown in FIG. 23, but with the plunger partially retracted;
FIG. 27 is the same view shown in FIG. 23 with the needle holder in the retracted position and the plunger in its fully advanced position;
FIG. 28 is a reduced longitudinal section of the barrel of the needle-syringe assembly of FIG. 22;
FIG. 29 is an end elevation of the barrel of FIG. 28;
FIG. 30 is a reduced side elevation of the guide sleeve in the needle-syringe assembly of FIG. 22;
FIG. 31 is an end elevation of the guide sleeve of FIG. 30;
FIG. 32 is an exploded view of the needle holder assembly in the needle-syringe assembly of FIG. 22;
FIG. 33 is an enlarged section of the distal end portion of the left-hand element of the needle holder assembly as shown in FIG. 32;
FIG. 34 is an enlarged section of the distal end portion of the barrel that receives the needle holder assembly of FIGs. 32 and 33;
FIG. 35 is an enlarged section of the distal end portion of a modified barrel for receiving a modified needle-holder assembly;
FIG. 36 is a fragmentary longitudinal section of the distal end portion of a modified needle holder assembly for use with the modified barrel shown in FIG. 35;
FIG. 37 is a side elevation of the plunger in the needle-syringe assembly of FIG. 22;
FIG. 38 is an end elevation of the distal end of the plunger of FIG. 37;
FIG. 39 is an end elevation of the proximal end of the plunger of FIG. 37;
FIG. 40 is a side elevation of a modified plunger for use in the needle-syringe assembly of FIG. 22;
FIG. 41 is an end elevation of the distal end of the plunger of FIG. 40;
FIG. 42 is an end elevation of the proximal end of the plunger of FIG. 40;
FIG. 43 is a side elevation, partially in section, of a modified needle-syringe assembly embodying the present invention;
FIG. 44 is the same view shown in FIG. 43 with the plunger partially retracted;
FIG. 45 is the same view shown in FIG. 43 with the needle and needle holder retracted and the plunger in its fully advanced position;
FIG. 46 is a side elevation, partially in section, of another modified needle-syringe assembly embodying the present invention;
FIG. 47 is an end elevation of the proximal end of the assembly shown in FIG. 46;
FIG. 48 is a side elevation of the latch mechanism included in the assembly of FIGs. 46 and 47;
FIG. 49 is an end elevation of the proximal end of another modified needle-syringe assembly embodying the invention;
FIG. 50 is a section taken generally along line 50-50 in FIG. 49;
FIG. 51 is a side elevation, partially in section, of a modified syringe embodying the invention;
FIG. 52 is an end elevation of the proximal end of the barrel in the syringe of FIG. 51, with the latching element removed;
FIG. 53 is a partial side elevation of a modified barrel and latch design;
FIG. 54 is a section taken generally along line 54-54 in FIG. 53;
FIG. 55 is a section taken generally along line 55-55 in FIG. 53;
FIG. 56 is a longitudinal section of a needle-syringe-guidewire assembly embodying the present invention;
FIG. 57 is a reduced side elevation, partially in section, of the assembly shown in FIG. 56;
FIG. 58 is the same view shown in FIG. 57, but with the plunger partially retracted;
FIG. 59 is the same view shown in FIG. 57 with the needle holder in the retracted position and the plunger in its fully advanced position;
FIG. 60 is an enlarged side elevation of the plunger and needle holder in the assembly of FIGs. 56-60;
FIG. 61 is an enlarged side elevation of the plunger in the assembly of FIGs. 56-60;
FIG. 62 is a section taken generally along line 62-62 in FIG. 61;
FIG. 63 is an end elevation of the plunger of FIG. 61;
FIG. 64 is a longitudinal sectional view of the external sleeve in the assembly of FIGs. 56-60;
FIG. 65 is a longitudinal sectional view of the barrel in the assembly of FIGs. 56-60, with the portion of the spiral slot in the removed portion of the barrel shown in broken lines;
FIG. 66 is a fragmentary longitudinal section of the portion of the barrel of FIG. 65 that receives the latch mechanism;
FIG. 67 is a section taken generally along line 67-67 in FIG. 66;
FIG. 68 is a longitudinal section of the valve assembly attached to the needle holder in the assembly of FIGs. 56-60, and a side elevation of the guide tubes attached to the valve assembly;
FIG. 69 is a separate longitudinal section of the valve elements of FIG. 68;
FIG. 70 is a separate longitudinal section of the valve housing of FIG. 68;
FIG. 71 is an end elevation of the housing of FIG. 70;
FIG. 72 is a separate side elevation of the outer guide tube shown in FIG. 68;
FIG. 73 is a separate side elevation of the inner guide tube of FIG. 68;
FIG. 74 is a side elevation, partially in section, of a modified needle-syringe-guidewire assembly embodying the present invention;
FIG. 75 is a section taken generally along lines 75-75 in FIG. 74;
FIG. 76 is a cross-sectional view similar to FIG. 75 but illustrating the external sleeve in two different positions, and with the cross-sectional hatching removed for clarity;
FIG. 77 is a side elevation, partially in section, of a modified needle-syringe-guidewire assembly embodying the present invention;
FIG. 78 is a section taken generally along lines 79-79 in FIG. 77;
FIG. 79 is a cross-sectional view similar to FIG. 78 but illustrating the open latch;
FIG. 80 is a perspective view of a needle-syringe assembly with guidewire embodying the present invention and is fully retracted;
FIG. 81 is a reduced perspective view of the same assembly shown in FIG. 80, with the needle in the advanced position;
FIG. 82 is a reduced perspective view of the same assembly shown in FIG. 1, with the needle in its advanced position and the plunger partially retracted; and
FIG. 83 is an exploded perspective of the needle-syringe assembly of FIGs. 80-82.

### Detailed Description Of The Preferred Embodiments

While the invention is susceptible to various modifications and alternative forms, specific embodiments thereof have been shown by way of example in the drawings and will herein be described in detail. It should be understood, however, that it is not intended to limit the invention to the particular forms disclosed, but on the contrary, the intention is to cover all modifications, equivalents, and alternatives falling within the scope of the invention as defined by the appended claims. Several different modes of the invention, each with its own unique features and alternate embodiments, are described. Permutations and combinations of these features will, however, lead to further modes.

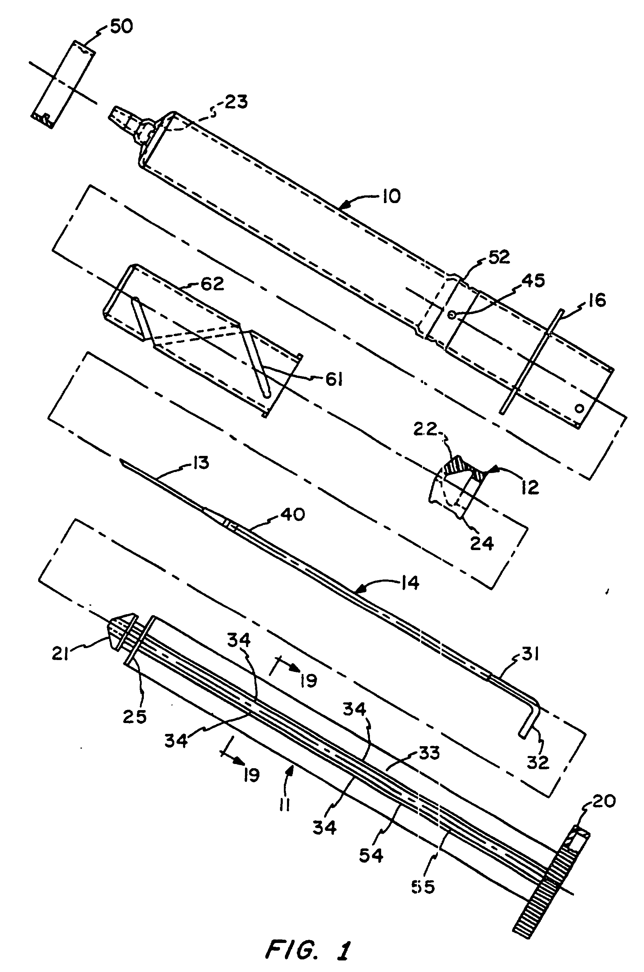

Turning now to the drawings, FIGs. 1-12 illustrate a needle-syringe assembly including a barrel 10, a plunger 11, a hollow plunger cap 12, a hypodermic needle 13, and a needle holder 14. The barrel 10 is a hollow cylinder which terminates in a hollow tapered nozzle 15 at the distal end thereof. The interior of the nozzle 15 communicates with the hollow interior of the tubular body portion of the barrel 10. The proximal portion of the barrel having the enlarged diameter provides a sufficient length to accommodate retraction of the needle holder 14 through a distance that is sufficient to draw the entire length of the needle 13 inside the barrel. An outwardly extending flange 16 near the proximal end of the barrel 10 facilitates gripping of the syringe with the user's fingers when it is desired to move the plunger 11 relative to the barrel 10.

The proximal end of the plunger 11 forms a knob 20 that can be grasped by a user to effect linear or rotary movement of the plunger 11 relative to the barrel 10. The periphery of the knob 20 is serrated to facilitate gripping of the knob for rotary movements of the plunger. The distal end of the plunger 11 forms a head 21 to accommodate the hollow rubber plunger cap 12. The outside diameter of the resilient cap 12 is reduced in the central portion so that the cap engages the inside wall of the barrel 10 only at the pliable margins of the ends of the cap. The diameter of the engaging end portions of the cap 12 is slightly larger than the inside diameter of the barrel 10 so that the cap presses firmly against the inside wall of the barrel to form an air-tight and liquid-tight seal at the cap/barrel interface. The inner margins of the cap 12 make a similar tight contact with the outer surface of the needle holder 14. The distal end 22 of the cap 12 is conical to conform to the conical distal end 23 of the inside surface of the barrel 10 when the plunger 11 is fully advanced within the barrel.

The head 21 of the plunger 11 is configured to fit tightly within the hollow plunger cap 12. With the cap 12 locked onto the head 21 of the plunger, the flat proximal end 24 of the cap abuts the flat surface of a circular disc 25 at the base of the plunger head 21. Due to the air-tight and liquid-tight seal between the plunger cap 12 and the barrel 10, as well as the needle holder 14, advancing movement of the plunger 11 inside the barrel 10 creates pressure in the interior of the barrel between the plunger cap and the distal end of the barrel. Similarly, retracting movement of the plunger 11 creates a vacuum in that portion of the barrel interior.

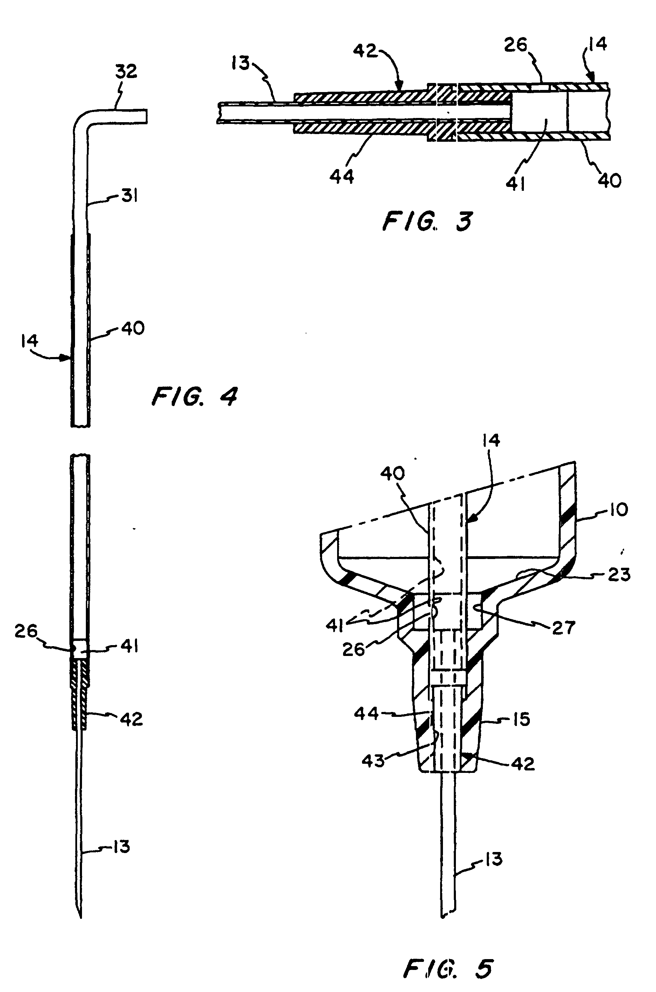

The hypodermic needle 13 projects from the distal end of the elongated needle holder 14, which is detachably interlocked to the barrel 10. Prior to use of the needle-syringe assembly, the needle 13 is covered by a protective cap (not shown) which prevents needle pricks and preserves sterility prior to use. Both the needle 13 and the needle holder 14 are hollow, and the interior of the hollow needle 13 communicates with the interior of the barrel 10 through an aperture 26 in the side walls of the needle 13 and the needle holder 14 (FIGs. 3 and 5). Prior to and during use of the needle-syringe assembly for withdrawal of blood and insertion of the guidewire (hereafter referred to as "normal use"), the aperture 26 is positioned at the base of the barrel nozzle 15 (FIG. 5), within a small cylindrical cavity 27. The aperture 26 permits blood to enter or exit from the barrel 10 via the needle 13 and the needle holder 14.

During normal use of the needle-syringe assembly, the needle holder 14 is locked to the barrel 10, and the plunger 11 and its cap 12 are free to slide longitudinally back and forth along the needle holder. The needle holder 14 includes a metal tube 40 and an L-shaped metal rod 30 having a longitudinal body portion 31 extending coaxially through the tube 40 within the barrel 10, and a lateral arm 32 extending radially across the barrel.

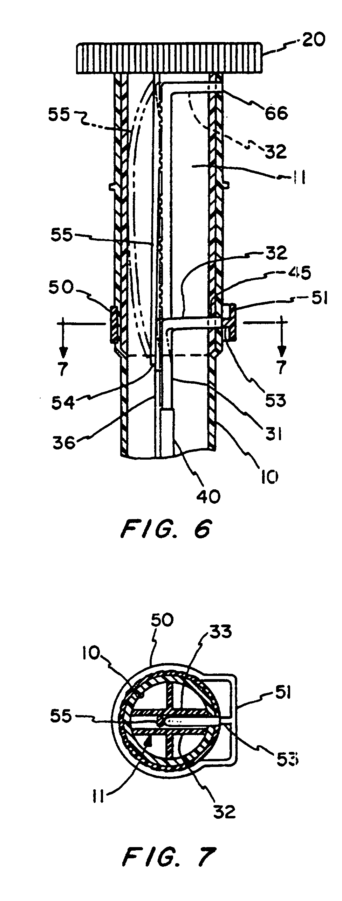

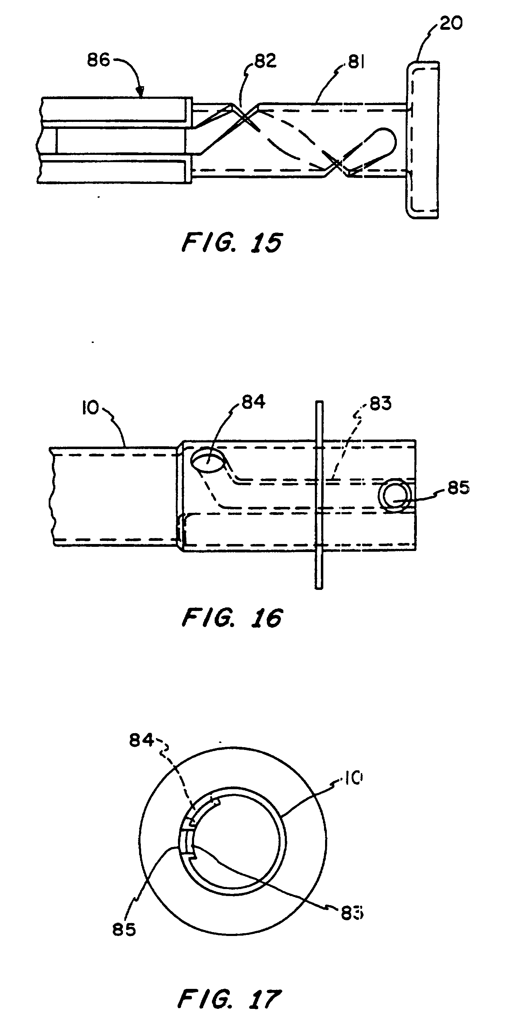

To permit relative sliding movement between the plunger 11 and the needle holder 14 in the longitudinal direction, the needle holder is mounted in a longitudinal channel 33 formed as an integral part of the plunger 11. Multiple pairs of resilient retaining elements 34 (FIG. 1) project toward each other from the opposed walls of the channel 33 to hold the needle holder 14 within the channel. These retaining elements 34 are deflected into adjacent recesses 35 during insertion of the needle holder 14 into the channel 33, and then the elements 34 spring back to their original positions (FIG. 15) after the needle holder is in place. It will be noted that the opposed walls of the channel 33 extend all the way to the inside wall of the barrel 10 (see FIG. 7), thereby constraining the lateral arm 32 of the needle holder against any angular or rotational displacement relative to the plunger 11. That is, the plunger 11 and the needle holder 14 can rotate only in unison with each other, although they are free to move independently of each other in the longitudinal direction. At the proximal end of the needle holder a pair of locking detents (described below) lock the arm and plunger after retraction is complete.

A major portion of the stainless steel rod 30 is encased in a hypodermic stainless steel tube 40 which extends beyond the distal end of the rod 30 and overlaps a portion of the needle 13 (see FIGs. 3-5). The opposed ends of the needle 13 and the rod 30 are separated slightly from each other, and the intervening space is surrounded by the stainless steel tube 40 to form a cavity 41 through which fluids pass between the hollow interiors of the needle 13 and the barrel 10 (see FIGs. 3-5). The aperture 26 mentioned previously is formed in this portion of the tube 40.

The distal end of the tube 40 abuts a shoulder on a plastic insert 42 bonded to that portion of the needle that is within the barrel nozzle 15. This insert 42 fits tightly against the inside surface of the nozzle 15, and these mating surfaces of the insert 42 and the nozzle 15 are tapered to from a conventional locking luer taper (typically 6% of the diameter). Specifically, the inside surface of the nozzle 15 forms a locking female luer taper 43, and the outside surface of the insert 42 forms a locking male luer taper 44. In one embodiment, the inside diameter of the nozzle 15 varies from 0,187 cm (0.0737 inch) at the proximal end of the taper to 0,157 cm (0.062 inch) at the distal end of the taper. The longitudinal distance between the two inside diameters is 0,476 cm (0.1875 inch). The diametric difference between the two diameters forms a taper in the nozzle 15 which is conventionally known as a locking female luer taper, and the angle formed by the diametric difference is conventionally known as a locking taper angle.

The locking surfaces 43 and 44 are engaged during the assembly of the needle syringe, when the plunger 11 and the needle holder 14 are inserted into the barrel 10 through the open distal end of the barrel. The resulting locking luer taper can be released only by the application of simultaneous axial and rotational forces. If desired, the tube 40 can be extended through the barrel nozzle and the taper formed on the distal end of the metal barrel rather on a plastic insert.

The proximal end of the needle holder 14 is also locked to the barrel 10, via the lateral arm 32 of the metal rod 30. As can be seen in FIGs. 6 and 7, this arm 32 extends radially beyond the plunger and fits into latching hole 45 in the barrel wall. This locking engagement of the arm 32 with the barrel wall can be released only by moving the arm 32 in a radial direction until the outer end of the arm clears the inside surface of the barrel wall.

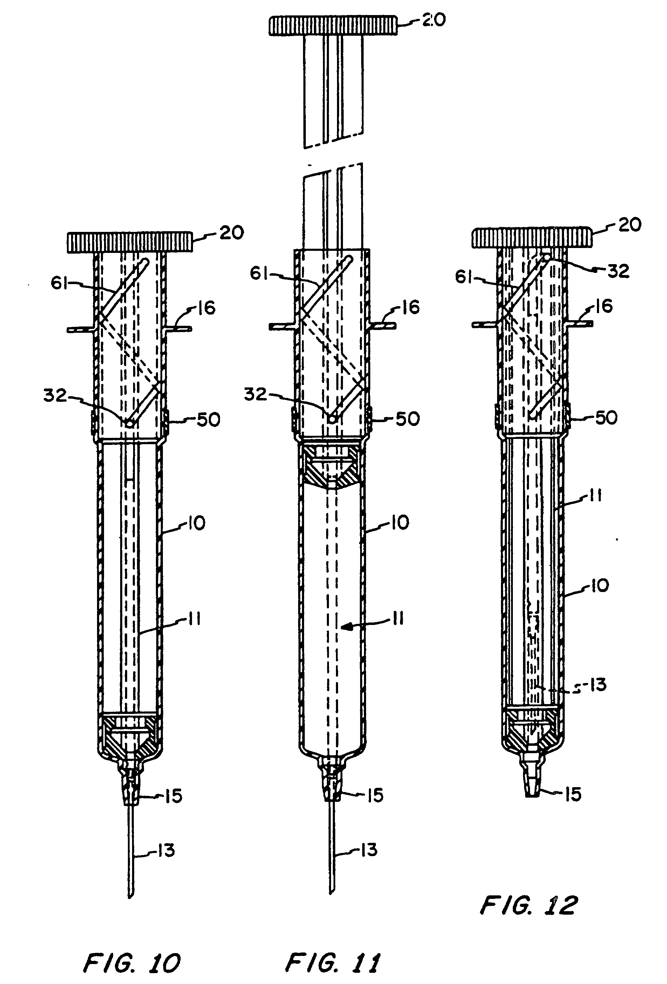

During normal use of the needle-syringe assembly, the barrel 10 and the needle holder 14 are held stationary and the plunger 11 is free to move relative to both the barrel 10 and the needle holder 14 (see FIGs. 10 and 11). Advancing movement of the plunger 11 is limited by contact of the plunger cap 12 with the end wall 23 of the barrel 10, as shown in FIG. 10. Retracting movement of the plunger is limited by contact of the plunger cap 12 with the arm 32. The needle holder 14 is locked to the barrel 10 by virtue of the taper lock between the distal portion of the needle holder and the barrel nozzle 15, and the locking engagement of the lateral arm 32 in the wall of the barrel. Alternatively, the needle holder can be locked to the nozzle by a threaded connection, as described in greater detail below. The plunger 11 is also free to move longitudinally relative to the needle holder 14, as illustrated in FIG. 11, because the needle holder is not locked to the plunger in that direction. The locking of the lateral arm 32 to the barrel wall prevents rotational movement of the plunger as well as the needle holder, and also prevents the plunger from being accidentally pulled out. As long as the lateral arm 32 of the needle holder is engaged with the barrel wall, the needle-syringe assembly is in its normal operating mode.

Following normal use of the needle-syringe assembly, the entire needle 13 can be retracted into the plunger 11 and the barrel 10. This requires axial movement of the needle holder 14 within the barrel 10 toward the proximal end thereof, which in turn requires that the needle holder 14 be unlocked from the barrel. Thus, to initiate retraction of the needle holder 14, the arm 32 is unlatched from the barrel 10 by pressing inwardly on a flat portion 51 of a resilient collar 50 captured in a groove 52 in the outer surface of the barrel. This causes a radial pin 53 extending inwardly from the flat portion 51 to enter the latching hole 45 in the barrel wall, engaging the outer end of the arm 32 and forcing it inwardly (see FIGs. 6 and 7). This can also be done by pushing directly on the arm with the user's finger, if a concave depression is formed around the latching hole 45, as described in more detail below. This inward force causes the body of the needle holder adjacent the arm 32 to retract, as illustrated in FIG. 6, which in turn moves the arm 32 inwardly far enough to clear the inner surface of the barrel wall. If desired, the opposed surfaces of the collar 50 and the barrel 10 may be provided with mating projections and recesses to hold the collar on the barrel, and the collar may even be adhesively bonded to the barrel.

To enable the rod 31 to disengage by moving laterally, the narrow wall 36 which forms the base of the channel 33 is cut transversely at 54 and then slit longitudinally along both edges from the cut 54 to a point just slightly spaced from the knob 20 (see FIG. 1). This transverse cut 54 and the longitudinal slits form a spring finger 55 which bears against the proximal end of the longitudinal portion of the rod 31 at all times, while permitting the rod 31 to be displaced laterally or transversely within the barrel 10.

While the flat portion of the collar 50 is pressed inwardly against the barrel 10 to release the arm 32 from the barrel wall, the plunger knob 20 is turned to rotate the plunger 11 counterclockwise (as viewed from the proximal end) relative to the barrel. As the plunger is rotated, the needle holder 14 rotates in unison with the plunger because the arm 32 is captured between the opposed parallel walls of the channel 32 in which the needle holder is mounted in the plunger. Rotation of the needle holder 14 relative to the barrel (1) retracts the needle holder within the plunger by the camming action of the spiral guide surface 60 acting on the arm 32, and (2) releases the locking luer taper at the distal end of the barrel nozzle 15 due to the resulting compound rotational and longitudinal forces applied to the tapered surfaces 43 and 44. As the rotation continues, the arm 32 traverses the entire length of the spiral surface 60, thereby retracting the entire needle holder 14 through the corresponding axial distance within the plunger 11 (see FIG. 12). Of course, the needle 13 is retracted along with the needle holder 14, and thus the needle is retracted completely within the barrel nozzle 10 and the plunger 11, as illustrated in FIG. 12.

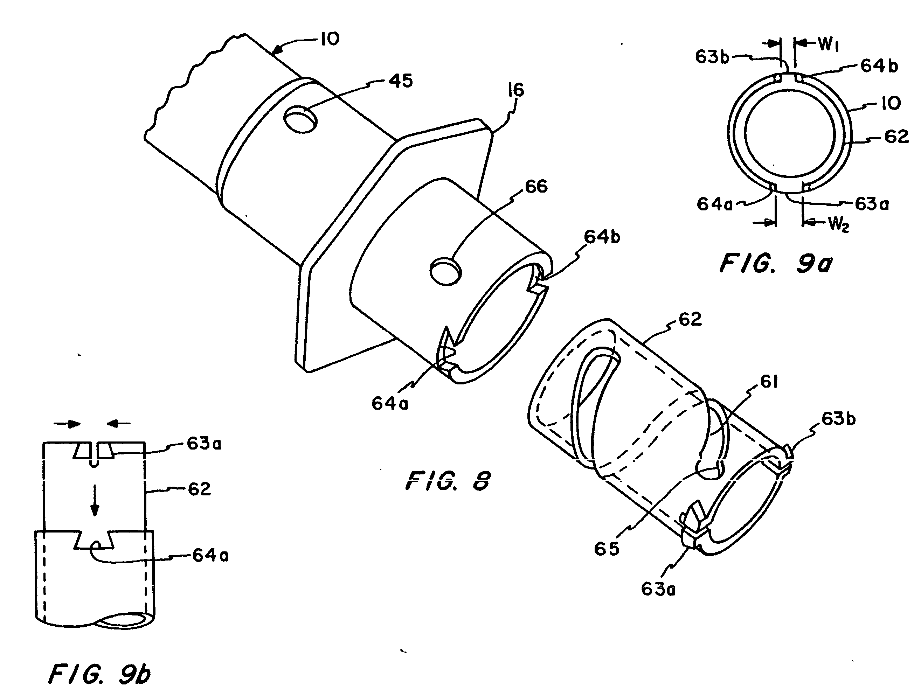

In this illustrative embodiment, the spiral guide surface 60 is formed by a spiral slot 61 in a sleeve 62 fitted inside the distal end portion of the barrel 10, and attached to the barrel. The illustration of the slot 61 is simplified in most of the drawings by the use of straight lines, but it will be understood that the spiral slot has a constant rate of curvature, as shown in FIG. 8. The portion of the barrel 10 that receives the sleeve 62 has a slightly larger diameter than the central portion of the barrel, and the sleeve 62 has the same inside diameter as the central portion of the barrel. Alternatively, a spiral channel can be molded as a part of the inside wall of the end portion of the barrel that has the slightly larger diameter.

To attach the sleeve 62 to the barrel 10, a pair of tabs 63a, 63b extend outwardly from opposite sides of the sleeve 62 into a pair of complimentary notches 64a, 64b in the distal end of the barrel. To lock the sleeve to the barrel longitudinally as well as rotationally, the tabs 63 and notches 64 have complimentary dovetail shapes. Each tab is bifurcated by a central slot so that the tapered sides of the tab can be displaced toward each other during the insertion of the tabs in the notches. Then when the tabs 63 resume their original shapes after being inserted into the notches 64, the two parts are securely locked together in both the longitudinal and circumferential directions.

To ensure that the spiral slot 61 in the sleeve 62 is always properly aligned with the locking holes in the barrel 10, the two tabs 63a, 63b and the two notches 64a, 64b have different widths, as can be seen in FIG. 9a. Thus, the locking hole 45 in the barrel 10 will always be aligned with the distal end of the spiral slot 61. The same effect may be achieved with the use of asymmetrical tabs of equal width.

Because the distal end of the spiral slot 61 is precisely registered with the latching hole 45 in the barrel wall, the arm 32 can pass through the slot 61 to gain access to the latching hole. When the arm 32 is disengaged from the latching hole 45, by manipulation of the collar 50 as described above, the arm still passes through the spiral slot 61, but the outer end of the arm now rides on the inside surface of the wall of the barrel 10 as the arm is cammed along the slot (see FIG. 6). The longitudinal portion of the rod 31 remains against the spring finger 55 during this movement of the arm 32 along the spiral slot 61. The retraction length is equal to the linear length of the spiral between the centers of the two holes 45 and 66.

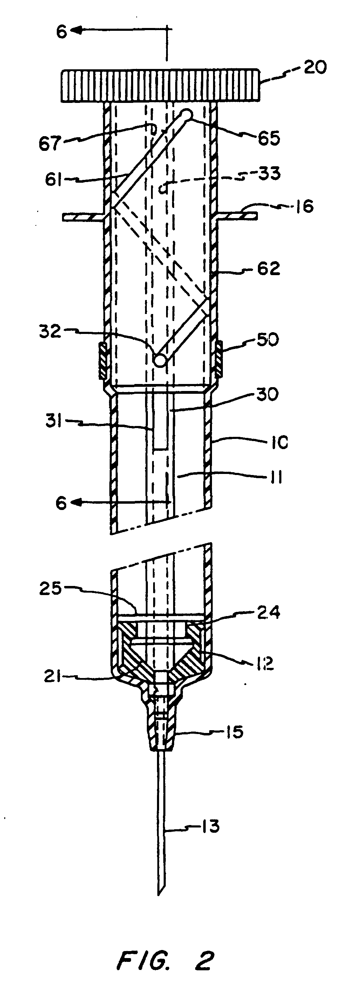

At the distal end of the spiral slot 61, the end of the arm 32 snaps into a detent notch 65 (FIGS. 2 and 8) formed by the walls of the slot so that the user feels the end of the needle retraction. At the same time, the outer end of the arm 32 enters a locking aperture 66 in the barrel wall. The arm 32 is forced into the locking aperture 66 by the inherent spring force of the rod itself, as well as the force of the spring finger 55, which increases toward the base of that finger. Then if the user attempts to turn the plunger knob 20 in the opposite direction, such attempt is met with firm resistance. This is a safety feature to prevent the needle from being returned beyond the end of the barrel nozzle, and to discourage re-use of the needle.

A pair of resilient locking fingers 67 are formed in the opposed walls of the channel 33 near the proximal end thereof to prevent the plunger from being withdrawn from the barrel 10 after the needle holder has been retracted. The arm 32 deflects the fingers 67 into adjacent recesses as the arm is retracted past the fingers, but the arm 32 then blocks any effort to retract the plunger over the needle holder.

To operate the needle-syringe assembly, the protective cap is removed from the needle 13, and the required amount of medication is aspirated into the barrel 10. Next, the injection site on the body of a patient is determined and the skin is cleaned with an antiseptic solution. Following percutaneous entry of the needle into the patient, location of the needle tip in the vein is confirmed by aspirating a small amount of blood into the transparent barrel 10. The plunger 11 is then advanced to force the medication from the barrel 10 into the vein. After the medication is administered, the needle 13 is withdrawn from the patient, the flat portion 51 of the collar 50 is pressed inwardly against the barrel 10, and the plunger knob 20 is rotated counterclockwise until the user feels the arm 32 snap into the locking aperture 66 and the detent 65 at the end of the spiral slot 61. The spiral slot 61 may alternatively be configured to require clockwise, instead of counterclockwise, rotation of the plunger knob 20. With the needle 13 completely retracted inside the barrel 10, the needle-syringe assembly can be safely discarded in its entirety.

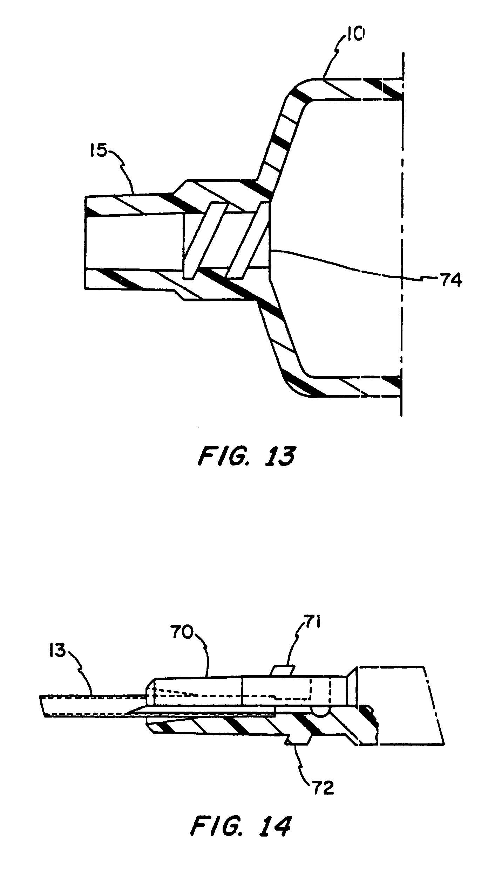

FIGS. 13 and 14 illustrate a modified construction for locking the distal end of the needle holder 14 to the barrel nozzle 15. In this design the plastic insert 42 is modified to form a pair of integral lugs 71 and 72 which thread into a threaded inside surface 73 in the barrel nozzle 15. When the plunger 11 is inserted into the barrel 10 and advanced to the distal end of the barrel, the distal end of the needle holder 14 carried in the plunger enters the nozzle 15. The plunger is automatically rotated by the advancing movement of the arm 32 through the spiral slot 61, and thus the lugs 71,72 are automatically threaded into the nozzle 15. Similarly, when the needle is subsequently retracted, the retracting movement of the arm 32 through the spiral slot 61, and the consequent rotation of the needle holder 14, threads the lugs 71,72 out of the barrel nozzle.

FIGS. 15-17 illustrate a modified plunger and barrel design in which the spiral guide surface is formed by the plunger rather than the barrel. Thus, the proximal end of the plunger 80 includes a hollow cylindrical section 81 adjacent the knob 20, and the wall of this section forms a spiral slot 82. The needle holder arm 32 extends radially outwardly through the slot 82 and into a generally linear channel 83 in the adjacent wall of the barrel 10. Latching holes 84 and 85 are formed in the barrel wall at opposite ends of the channel 83. When the arm 32 is released from the forward latching hole 82, the plunger is turned to move the end of the arm 32 into the channel 83. The arm 32 moves through the hole 82 until it engages the side of the longitudinal section of the channel 83, which then permits longitudinal but not rotational movement of the needle holder as the plunger continues to be turned. Thus, the spiral slot 82 in the plunger acts as a camming surface to urge the arm 32 along the channel 83, thereby retracting the needle 13 into the barrel 10 and ultimately locking it to the barrel margin as described for the first embodiment.

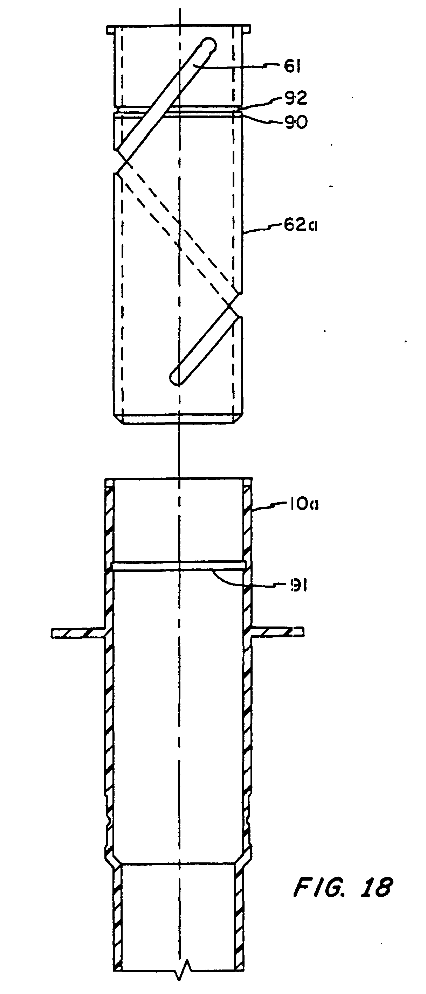

FIG. 18 illustrates a modified design for locking the barrel 62 to the barrel 10. In this case the outside wall of the barrel sleeve 62a forms a resilient lip 90 which snaps into a complementary groove 91 in the inside wall of the barrel body 10a. During sliding movement of the sleeve 62a through the barrel 10a, the lip 90 is bent into an adjoining groove 92 in the sleeve. The interlocking of the lip 90 and the groove 91 prevent relative longitudinal movement between the sleeve and the barrel. A locking tab, of the type described above, can be provided to lock the two members against rotational movement.

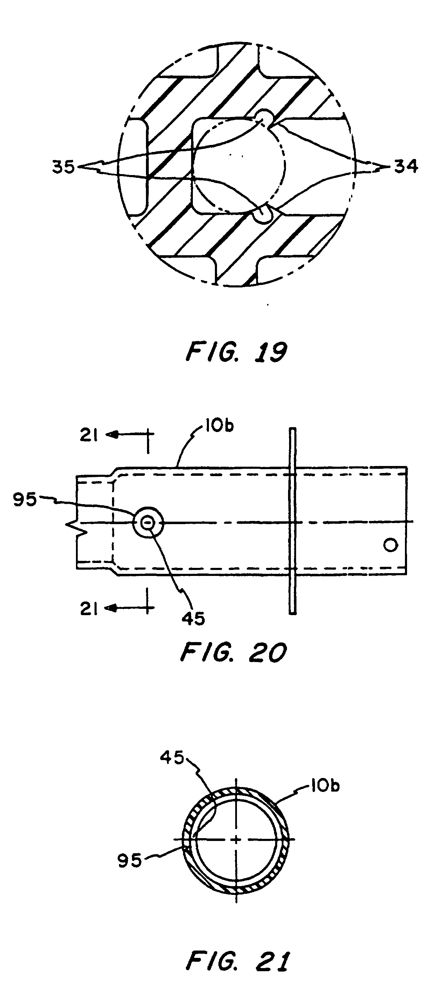

FIGS. 20 and 21 show a modified design for the proximal end of the barrel 10. This modified barrel 10b eliminates the need for the collar 50 by providing a circular depression around the latching hole 45. This depression 95 allows a user's finger to engage the end of the lateral arm 32 and to press the arm inwardly to disengage it from the latching hole 45. The plunger is then turned relative to the barrel while the arm 32 is pressed inwardly, to initiate the retracting movement of the needle holder.

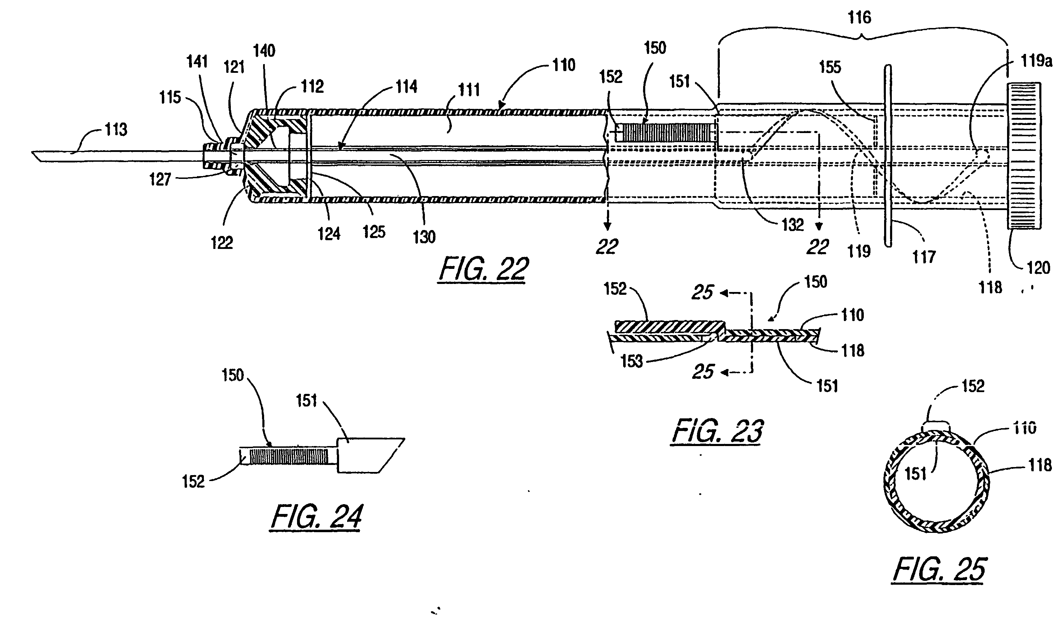

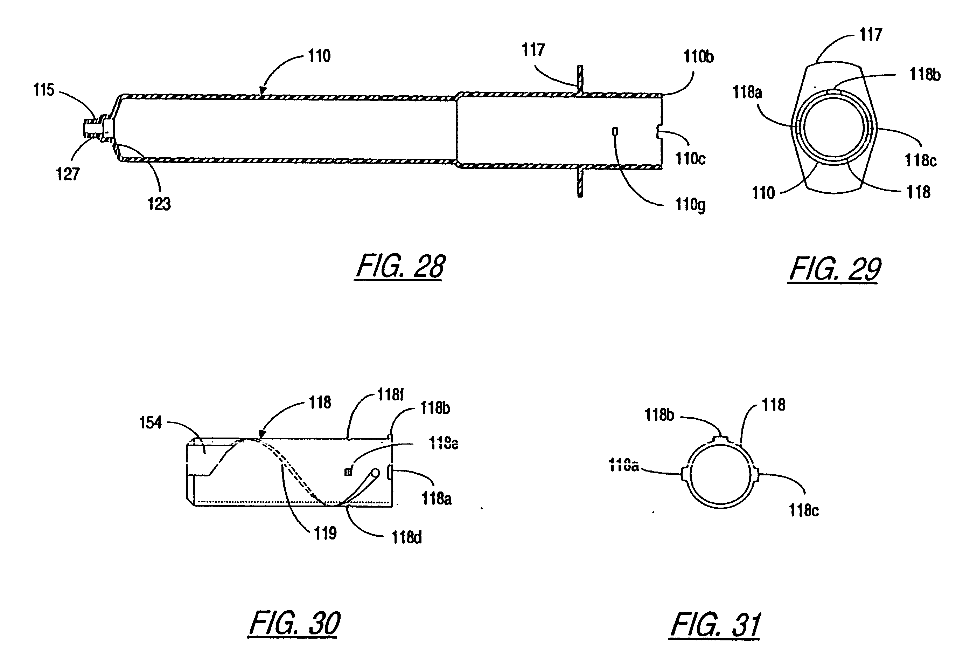

Turning now to FIGs. 22-34 and 37-39 another needle-syringe assembly is illustrated including a barrel 110, a plunger 111, a hollow plunger cap 112, a hypodermic needle 113, and a needle holder 114. The barrel 110 is a hollow cylinder which terminates in a hollow tapered nozzle 115 at the distal end thereof, and has a slightly enlarged diameter along a proximal end portion 116. The interior of the nozzle 115 communicates with the hollow interior of the tubular body portion of the barrel 110. An outwardly extending flange 117 near the proximal end of the barrel 110 facilitates gripping of the barrel with the user's fingers when it is desired to move the plunger 111 relative to the barrel 110.

The purpose of the enlarged diameter of the proximal end portion 116 of the barrel 110 is to accommodate a sleeve 118 incorporating a spiral slot 119 within its wall and without encroaching on the inner diameter of the entire syringe. As will be described below, this spiral slot 119 provides an internal retraction track for the needle holder 114 and the hypodermic needle 113. The sleeve 118 and its spiral slot 119 extend along a sufficient length to accommodate retraction of the needle holder 114 through a distance that is sufficient to draw the entire length of the needle 113 inside the barrel 110, as described in more detail below. The outer surface of the barrel 110 preferably contains graduations (not shown) indicating the volume level of fluid in the barrel. These graduations take into account the volume of the internal components such as the needle holder 114.

The proximal end of the plunger 111 forms a knob 120 that can be grasped by a user to effect linear or rotary movement of the plunger 111 relative to the barrel 110. The periphery of the knob 120 is serrated to facilitate gripping of the knob for rotary movements of the plunger. The distal end of the plunger 111 forms a head 121 to accommodate the hollow rubber plunger cap 112. The outside diameter of the resilient cap 112 is reduced in the central portion so that the cap engages the inside wall of the barrel 110 only at the pliable margins of the ends of the cap. The diameter of the engaging end portions of the cap 112 is slightly larger than the inside diameter of the barrel 110 so that the cap presses firmly against the inside wall of the barrel to form an air-tight and liquid-tight seal at the cap/barrel interface. The inner margins of the cap 112 make a similar tight contact with the outer surface of the needle holder 114. The distal end 122 of the cap 112 is conical to conform to the conical distal end 123 of the inside surface of the barrel 110 when the plunger 111 is fully advanced within the barrel.

The head 121 of the plunger 111 is configured to fit tightly within the hollow plunger cap 112. With the cap 112 locked onto the head 121 of the plunger, the flat proximal end 124 of the cap abuts the flat surface of a circular disc 125 at the base of the plunger head 121. Due to the air-tight and liquid-tight seal between the plunger cap 112 and the barrel 110, as well as the needle holder 114, advancing movement of the plunger 111 inside the barrel 110 creates pressure in the interior of the barrel between the plunger cap and the distal end of the barrel. Similarly, retracting movement of the plunger 111 creates a vacuum in that portion of the barrel interior.

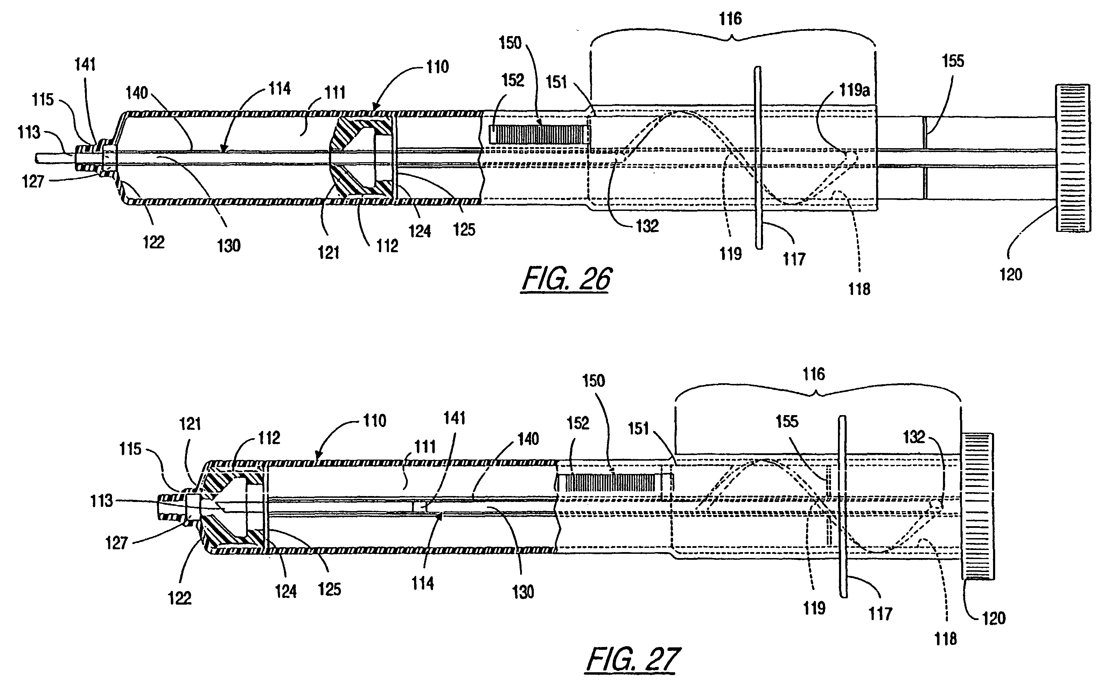

The hypodermic needle 113 is mounted on the distal end of the elongated needle holder 114, which is detachably interlocked to the barrel 110. Prior to use of the needle-syringe assembly, the needle 113 is covered by a protective cap (not shown) which prevents needle pricks and preserves sterility prior to use. Both the needle 113 and the distal portion of the needle holder 114 are hollow, and the interior of the hollow needle 113 communicates with the interior of the hollow distal portion of the needle holder 114. The needle holder 114 further communicates with the interior of the barrel 110 through an aperture 126 in the side wall of the hollow portion of the needle holder 114 (FIGs. 25 and 27). Prior to and during use of the needle-syringe assembly for injection of medicine or withdrawal of blood (hereafter referred to as "normal use"), the aperture 126 is positioned at the base of the barrel nozzle 115 (FIG. 27), sometimes within a small cylindrical cavity 127. The aperture 26 permits blood or medicine to enter or exit from the barrel 110 via the needle holder 114 and the needle 113.

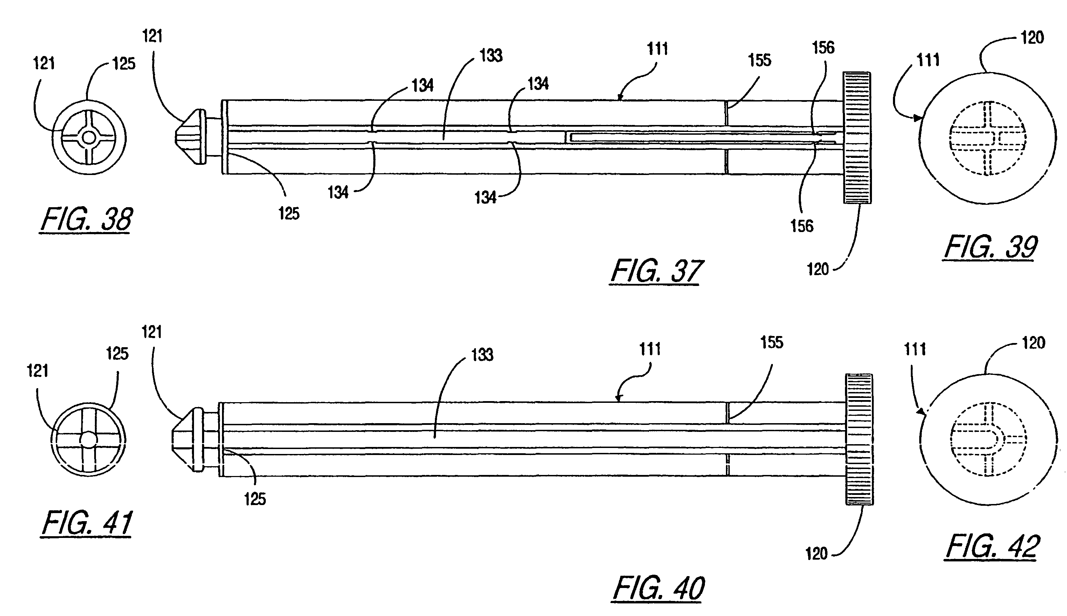

During normal use of the needle-syringe assembly, the needle holder 114 is locked to the barrel 110, and the plunger 111 and its cap 112 are free to slide longitudinally back and forth along the needle holder. The needle holder includes a metal tube 140 an L-shaped metal rod 130 having a longitudinal body portion 131 extending coaxially through the tube 140 to the aperture 126, and the lateral arm 132 extending radially across the barrel 110 to the proximal end of the rod 130. Multiple pairs of resilient retaining elements 134 (FIG. 37) project toward each other from the opposed walls of channel 133 to hold the needle holder 114 within the channel. These retaining elements 134 are deflected into adjacent recesses during insertion of the needle holder 114 into the channel 133, and then the elements 134 spring back into their original positions after the needle holder is in place. It will be noted that the opposed walls of channel 133 extend all the way to the inside wall of the barrel 110 (see FIG. 39), thereby constraining the lateral arm 132 of the needle holder against any angular or rotational displacement relative to the plunger 111. That is, the plunger 111 and the needle holder 114 can rotate only in unison with each other, although they are free to move independently of each other in the longitudinal direction. At the proximal end of the needle holder, a locking detent (described below) locks the arm and plunger together to prevent relative longitudinal movement after retraction is complete.

A major portion of the stainless steel rod 130 is encased in stainless steel hypodermic tube 140 which extends beyond the distal end of the rod 130 and overlaps a portion of the needle 113. The opposed ends of the needle 113 and the rod 130 are separated slightly from each other, and the intervening space is surrounded by the stainless steel tube 140 to form a cavity 141 through which fluids pass between the hollow interiors of the needle 113 and the barrel 110. The aperture 126 mentioned previously is formed in this portion of the tube 140.

To lock the needle holder 114 to the barrel 110, the outer surface of the distal end portion of the metal cover tube 140 is ground (e.g., by centerless grinding) to form a tapered surface 140a which mates with a complementary tapered surface 115a on the inside wall of the barrel nozzle 115. These tapered surfaces 140a and 115a are locking luer tapers, and the angle of the taper (typically 6% of the diameter) is conventionally known as a locking taper angle. In one embodiment, the taper has a length between about 0,476 and 0,635 cm (0.185 and about 0.250 inch) with a diameter of 0,239 cm (0.094 inch) at one end and a diameter of 0,21 cm (0.082 inch) at the other end.

The locking surfaces 115a and 140a are engaged during assembly of the needle syringe, when the plunger 111 and needle holder 114 are inserted into the barrel 110 through the open proximal end of the barrel. The resultant locking luer taper can be released only by the application of simultaneous axial and rotational forces.

The proximal end of the needle holder 114 is also locked to the barrel 110, via the lateral arm 132 of the metal rod 130. This arm 132 extends radially beyond the plunger and fits into the spiral slot 119 in the sleeve 118. The arm 132 can be locked to the barrel 110 at either end of the spiral slot 119 and, when so locked, permits only reciprocal linear movement of the plunger 111, to create vacuum to withdraw medication or blood and pressure to deliver medication to the patient via the hypodermic needle. When the arm 132 is locked at either end of the slot 119, the plunger 111 cannot be rotated within the barrel 110.

When the user desires to retract the hypodermic needle 113 within the barrel-plunger assembly, a mechanical latch 150 is manually actuated to unlock the arm 132 and thereby permit rotation of the plunger 111 relative to the barrel 110. This relative rotation retracts and locks the needle-needle holder assembly within the barrel-plunger assembly. For the needle and needle holder to be moved to the retracted position, the plunger 111 can be in any desired position, e.g., to permit blood or medication to be retained in the syringe.

A latch mechanism 150 of FIGs. 22-27 includes an arcuate locking plate 151 and an integral handle 152 mounted for sliding movement within a short longitudinal slot 153 in the wall of the barrel 110. The free end of the plate 151 is angled to match the slope of the side walls of the spiral slot 119, and the plate slides back and forth within a slot 154 formed in the sleeve 118 and opening into the slot 119 adjacent the distal end of the slot. The inner and outer radii r1 and r2 of the plate 151 match those of the sleeve 118 (see FIG. 23) to ensure that the locking plate 151 fits precisely into the slot 154 in the sleeve 118 and cannot fall inside the barrel cavity to obstruct movement of the plunger 111, in either the locked or unlocked position of the latch. As can be seen in FIG. 23, the locking plate 151 and the handle 152 are offset from each other in the radial direction so that the handle rides on the outer surface of the barrel 110. This outer handle surface is serrated to facilitate movement thereof with the user's finger or thumb.

The latch 150 can be opened or closed by linear movement of the locking plate 151 via the handle 152. During normal use, the needle holder arm 132 is positioned at the distal end of the spiral slot 119 and the locking plate 151 is advanced into the spiral slot 119 to close the slot and retain the arm 132 at the distal end of the slot 119. This locks the needle holder 114 in the normal operative mode in which only linear reciprocal movement of the plunger 111 is permitted. Because the locking plate 151 blocks the spiral slot 119, the needle holder 114 cannot rotate and thus cannot travel along the spiral slot 119 for retraction of the hypodermic needle 113. When it is desired to retract the needle, the latch handle 152 is retracted toward the distal end of the syringe, thereby opening the spiral slot 119 and permitting rotation of the plunger 111 and retraction of the needle holder 114 by movement of the arm 132 along the spiral slot.

It will be appreciated that when the latch 150 is retracted to open the spiral slot 119 and thereby unlock the arm 132, the plunger can be in any desired longitudinal position. That is, the plunger can be fully advanced, fully retracted, or at any intermediate position. This is advantageous because it might be desired to retract the needle after only a portion of a dose of medication has been injected into the patient, or it might be desired to retain a portion of a blood sample withdrawn from a patient within the syringe. To prevent the leakage of any fluid contained within the syringe at the time the needle is retracted, it is preferred to provide a latex seal (not shown) at the end of the nozzle 115.

To ensure retention of the end portion of the arm 132 within the spiral slot 119 during retracting movement of the needle holder 114, the plunger 111 includes an integral circular retaining plate 155. The diameter of this plate 155 matches the inside diameter of the guide sleeve 118 so that it tends to maintain the desired circular shape of the inside wall of the sleeve 118. Stresses exerted on the wall of the barrel during use can tend to distort the desired circular configuration of the sleeve 118, and if the distortion becomes large enough, the arm 132 can escape from the spiral slot 119. With the retainer plate 155 riding on the inside wall of the sleeve 118, however, such excessive distortion is prevented, and thus retention of the arm 132 within the spiral slot 119 is ensured. Of course, in addition to the retainer plate 155, the longitudinal ribs of the plunger also glide on the inside wall of the sleeve 118 at approximately 90° intervals from each other, and thus further ensure that the sleeve 118 retains its desired circular configuration.

During normal use of the needle-syringe assembly, the barrel 110 and the needle holder 114 are held stationary, and the plunger 111 is free to move relative to both the barrel 110 and the needle holder 114. Advancing movement of the plunger 111 is limited by contact of the plunger cap 112 with the end wall of the barrel 110, as shown in FIG. 22. Retracting movement of the plunger 111 is limited by contact of the plunger disc 125 with the arm 132. If desired, stop members may be provided on the inside surface of the barrel to engage the disc 125 on the distal side of the latch opening, to further protect against the leakage of fluids through the latch opening in the barrel wall. The needle holder 114 is locked to the barrel 110 by virtue of the taper lock between the distal portion of the needle holder and the barrel nozzle 115, and the locking engagement of the lateral arm 132 in the wall of the barrel. Alternatively, the needle holder can be locked to the nozzle by a threaded connection. The plunger 11 is also free to move longitudinally relative to the needle holder 114, as illustrated in FIG. 26, because the needle holder is not locked to the plunger in that direction. The locking of the lateral arm 132 to the barrel wall prevents rotational movement of the plunger as well as the needle holder, and also prevents the plunger from being accidentally pulled out. As long as the lateral arm 132 of the needle holder is locked to the barrel wall, the needle-syringe assembly is in its normal operating mode.

Following normal use of the needle-syringe assembly, the needle 113 can be retracted into the plunger 111 and the barrel 110. This requires axial movement of the needle holder 114 within the barrel 110 toward the proximal end thereof, which in turn requires that the needle holder 114 be unlocked for movement along the spiral slot 119. Thus, to initiate retraction of the needle holder 114, the arm 132 is unlocked by retracting the locking plate 151.

After the latching plate 151 has been retracted, the plunger knob 120 is turned to rotate the plunger 111 clockwise (as viewed from the proximal end) relative to the barrel. As the plunger is rotated, the needle holder 114 rotates in unison with the plunger because the arm 132 is captured between the opposed parallel walls of the channel 132 in which the needle holder is mounted in the plunger. Rotation of the needle holder 114 relative to the barrel (1) retracts the needle holder within the plunger by the camming action of the wall of the spiral slot 119 acting on the arm 132, and (2) releases the locking luer taper at the distal end of the barrel nozzle 115 due to the resulting compound rotational and longitudinal forces applied to the tapered surfaces 115a and 140a. As rotation continues, the arm 132 traverses the entire length of the spiral slot 119, thereby retracting the entire needle holder 114 through a corresponding axial distance within the plunger 111 (see FIG. 27). Of course, the needle 113 is retracted along with the needle holder 114, and thus the needle is retracted completely within the barrel nozzle 110 and the plunger 111, as illustrated in FIG. 27.

In an illustrative embodiment, the spiral slot 119 is formed in a sleeve 118 fitted inside a distal end portion of the barrel 110, and attached to the barrel. The spiral slot preferably has a constant rate of curvature along its length. The portion of the barrel 110 that receives the sleeve 118 has a slightly larger diameter than the central portion of the barrel, and the sleeve 118 has the same inside diameter as the central portion of the barrel. Alternatively, a spiral channel can be molded as a part of the inside wall of the end portion of the barrel that has the slightly larger diameter. The illustrative syringe need not be any longer than a conventional syringe because conventional syringes are made longer than required to provide more than the desired fluid volume, so as to avoid inadvertent withdrawal of the plunger and the resultant spillage of the syringe contents. The extra barrel length also accommodates the user's fingers in the space between the plunger knob and the finger flanges. The present invention permits the extension of the barrel length in this area to be used for the needle-retracting mechanism.

To attach the sleeve 118 to the barrel 110, three tabs 118a, 118b, 118c extend radially outwardly from the sleeve 118 into complementary notches 110a, 110b, 110c in the distal end of the barrel. To lock the sleeve to the barrel longitudinally as well as rotationally, four fingers 118d - 118g on opposite sides of the sleeve 118 snap into complementary recesses 110d - 110g on the inside wall of the barrel.

At the distal end of the spiral slot 119, the end of the arm 132 snaps into a detent notch 119a (FIGs. 22, 26 and 27) formed by the walls of the slot so that the user feels the end of the needle retraction, as a click. Then if the user attempts to turn the plunger knob 120 in the opposite direction, such attempt is met with firm resistance. This is a safety feature to prevent the needle from being returned beyond the end of the barrel nozzle, and to discourage re-use of the needle.

A pair of resilient locking fingers 156 (FIG. 37) are formed in the opposed walls of the channel 133 near the proximal end thereof to prevent the plunger 111 from being withdrawn from the barrel 110 after the needle holder 114 has been retracted. The arm 132 deflects the fingers 156 into adjacent recesses as the arm is retracted past the fingers, but the arm 132 then blocks any effort to retract the plunger 111 over the needle holder 114.

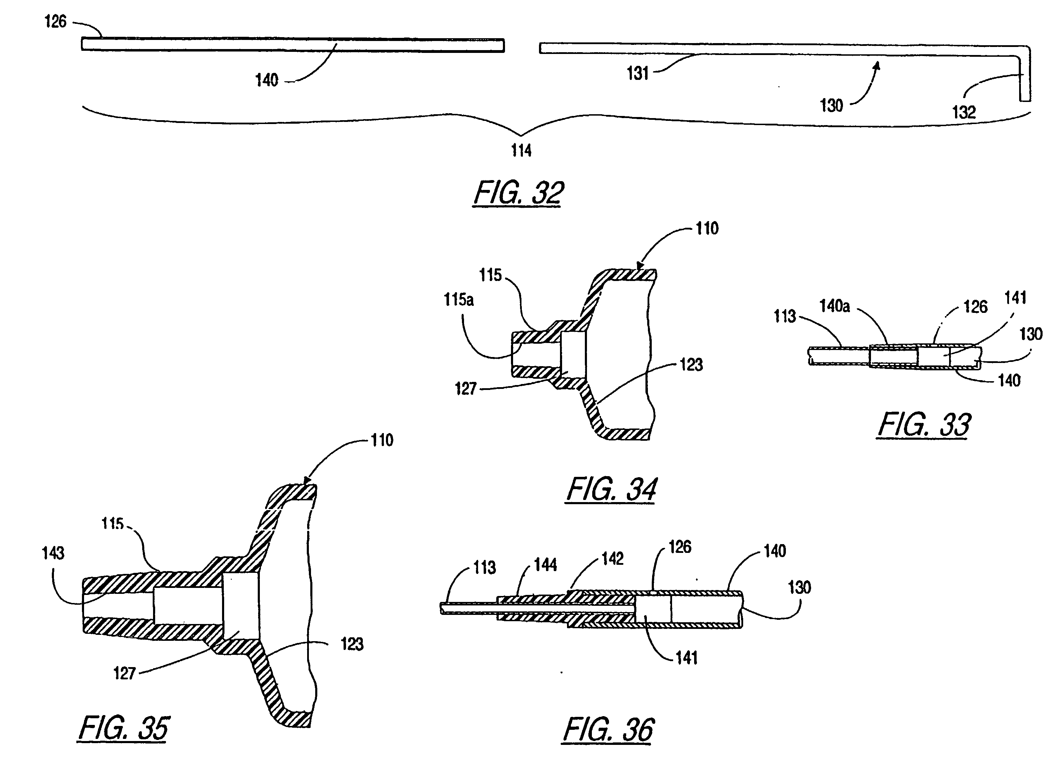

FIGs. 40-42 illustrate a modified plunger for use with a needle holder having a larger diameter than the needle holder used with the plunger of FIGs. 37-39. For example, the relatively thin needle holder of FIG. 32 can be used in the plunger of FIGs. 37-39, and the larger-diameter needle holder of FIG. 36 can be used in the plunger of FIGs. 40-42. It will be noted that the passageway 133 is wider in the plunger of FIGs. 40-42 than in the plunger of FIGs. 37-39. Similarly, the hole that extends through the distal end of the plunger is also larger in FIG. 41 than it is in FIG. 38. Certain of the details of the plunger of FIGs. 37-39, such as the retaining elements 134 and the fingers 156, have not been included in the illustration of the plunger in FIGs. 40-42, but it will be understood that similar features may be included in both plungers.

To operate the needle-syringe assembly, the protective cap (not shown) is removed from the needle 113, and the required amount of medication is aspirated into the barrel 110. Next, the injection site on the body of a patient is determined and the skin is cleaned with an antiseptic solution. Following percutaneous entry of the needle into the patient, location of the needle tip in the vein is confirmed by aspirating a small amount of blood into the transparent barrel 110. The plunger 111 is then advanced to force the medication from the barrel 110 into the vein. After the medication is administered, the needle 113 is withdrawn from the patient, the latch handle 152 is retracted to open the spiral channel 119, and the plunger knob 120 is rotated clockwise until the user feels the arm 132 snap into the detent notch 119a at the proximal end of the spiral slot 119. The spiral slot 119 may alternatively be configured to require counterclockwise, instead of clockwise, rotation of the plunger knob 120. With the needle 113 completely retracted inside the barrel 110, the needle-syringe assembly can be safely discarded in its entirety.

FIGs. 35 and 36 illustrate a modified construction for locking the distal end of the needle holder 114 to the barrel nozzle 115. In this design the distal end of the tube 140 abuts a shoulder on a plastic insert 142 bonded to that portion of the needle that is within the barrel nozzle 115. This insert 142 fits tightly against the inside surface of the nozzle 115, and these mating surfaces of the insert 142 and the nozzle 115 are tapered to form a conventional locking luer taper (typically 6% of the diameter). Specifically, the inside surface of the nozzle 115 forms a locking female luer taper 143, and the outside surface of the insert 142 forms a locking male luer taper 144. In one embodiment, the inside diameter of the nozzle 115 varies from 0,187 cm (0.0737 inch) at the proximal end of the taper to 0,157 cm (0.0625 inch) at the distal end of the taper. The longitudinal distance between these two inside diameters is 0,476 cm (0.1875 inch). The diametric difference between the two diameters forms a locking luer taper in the nozzle 115.

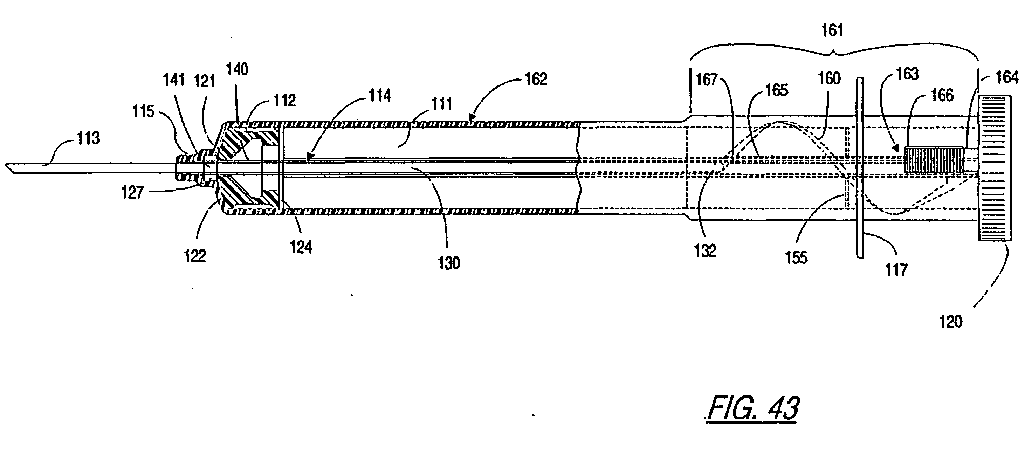

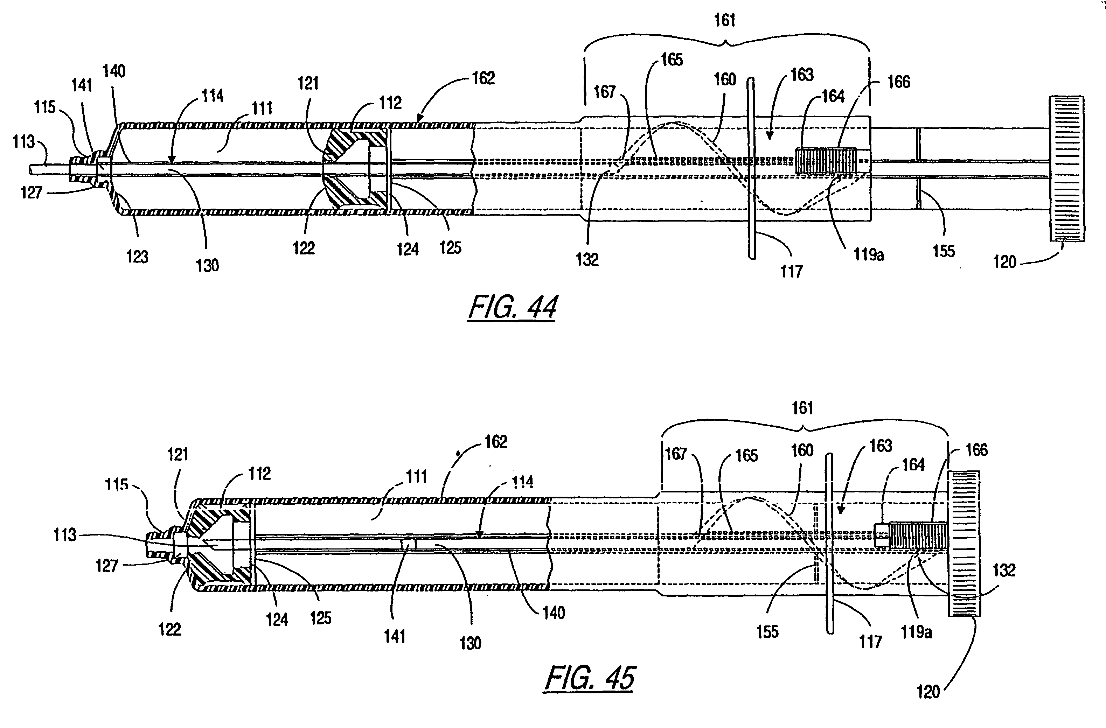

A modified embodiment and latch mechanism is shown in FIGs. 43-45. In this embodiment, a spiral channel 160 is integrally molded on the inner surface of a proximal end portion 161 of a barrel 162. To accommodate the spiral channel 160, the wall thickness of the barrel 162 is increased in the end portion 161. Internal molding of the spiral channel 160 is possible when mold cores are rotated while they are pulled from the mold cavity used to form the barrel 162. A latch mechanism 163 is mounted in a straight longitudinal slot 164 that opens through the proximal end of the barrel 162 and continues as a straight longitudinal channel 165 on the inside wall of the barrel 162. The spiral channel 160 preferably extends less than 360 degrees around the circumference of the barrel 162, and opens through the proximal end of the barrel. This avoids interference between the spiral slot 160 and the channel 165. The slot 164 receives a serrated latch handle 166 that fits precisely into the slot 164 but is shorter than the slot in the axial direction and mates with recesses in the side walls of the slot 164. The handle 166 is molded on a metal pin 167 that extends distally beyond the slot 164 and along the entire length of the channel 165 which opens into the end portion of the spiral channel 160 adjacent the distal end thereof. The external surface of the handle 166 is serrated to facilitate frictional gripping for capture and release of the needle holder arm by the latching pin 167.

During assembly, the right-angle needle holder arm 132 is engaged in the spiral channel 160 through its open end, and then the end of the spiral channel is closed by heat crimping. The needle holder 114 is advanced until the arm 132 reaches the distal end of the spiral channel 160. At this location the needle holder arm 132 is locked in its advanced position in the spiral channel 160 by advancing the handle 166 so that the pin 167 enters and blocks the special channel 60. This prevents rotation of the needle holder 114 so that it cannot be moved along the spiral channel 160. Linear movements of the plunger 111 do not affect the pin engagement that is perpendicular to the spiral. When it is desired to retract the needle 113, the handle 166 is retracted to the proximal end of the barrel 162 to retract the pin 167 and open the spiral channel 160, thereby permitting rotation of the plunger 111 and resultant retraction of the needle holder 114 via the spiral channel.

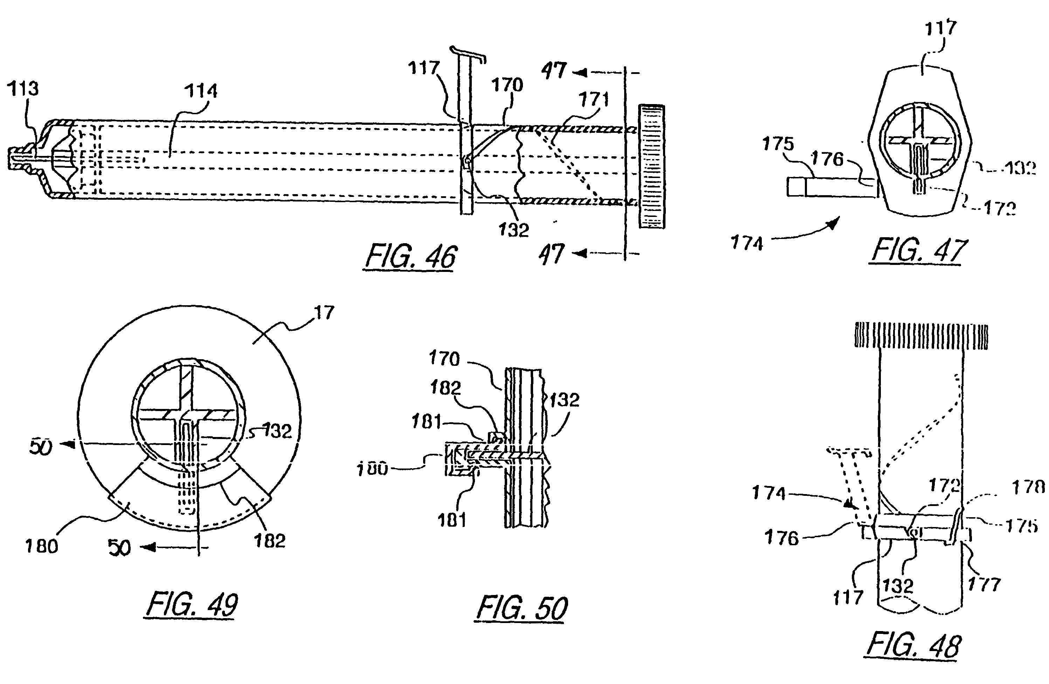

Yet another modified embodiment and latch mechanism is shown in FIGs. 46-48. In this embodiment, the needle holder 114 with a relatively short hypodermic needle 113 is engaged in a barrel 170 having the normal finger flange 117 and extended barrel length to reach the plunger knob 120. In this embodiment, the spiral track for retracting movement of the arm 132 is formed by a spiral slot 171 which extends through the entire thickness of the barrel wall. It will also be noted that the barrel 170 has a uniform wall thickness, and a uniform outside diameter, along its entire length, including that portion of the barrel in which the spiral slot 171 is formed and extends well beyond the finger flange to the plunger knob. This greatly facilitates molding of the barrel, including the spiral slot 171, without the use of any special technique. It will be appreciated that the position of the finger flange and the length of the barrel extension on the proximal side of the flange can be varied as required to retract needles of different lengths.

The distal end of the spiral slot 171 terminates within the thickness of the finger flange 117. Moreover, the radial length of the needle holder arm 132 is extended so that the end of the arm 132 projects beyond the outer surface of the barrel 170 and into a groove 172 formed in the flange 117, as a continuation of the distal end portion of the spiral channel 171 formed in the wall of the barrel. Consequently, when the arm 132 is positioned at the distal end of the spiral slot 171, the radially outer end portion of the arm 132 is positioned in the groove 172 formed in the flange 117. This permits the use of a latch mechanism mounted on, or formed as a part of, the finger flange 117. A bead of resin may be glued to the end of the needle holder arm 132 projecting beyond the barrel wall to ensure that the arm remains engaged in the spiral slot 171.

In the particular embodiment illustrated in FIGs. 47 and 48, a latch 174 is molded as an integral part of the finger flange 117. The latch 174 includes an arm 175 connected to the flange 117 by a thin hinge 176 so that the arm 175 can be pivoted over the flange 117 to clamp the needle holder arm 132 against the flange 117. An L-shaped extension 177 on the free end of the arm 175 snaps over the flange 117 (see FIG. 48) to hold the arm 175 in its closed position until it is released by pressing on a rib 178 projecting from the opposite side of the arm 175. This is an advantageous construction in that it permits the latch to be molded as an integral part of the barrel 170.

An alternative latch construction for use with a circular finger flange is shown in FIGs. 49 and 50. In this embodiment, a separately molded, U-shaped latch element 180 fits over the portion of the flange that contains the end portion of the needle holder arm 132. A series of detents 181 formed in the mating surfaces of the circular flange, the barrel surface and the latch element 180 serve to hold the latch in position on the flange.

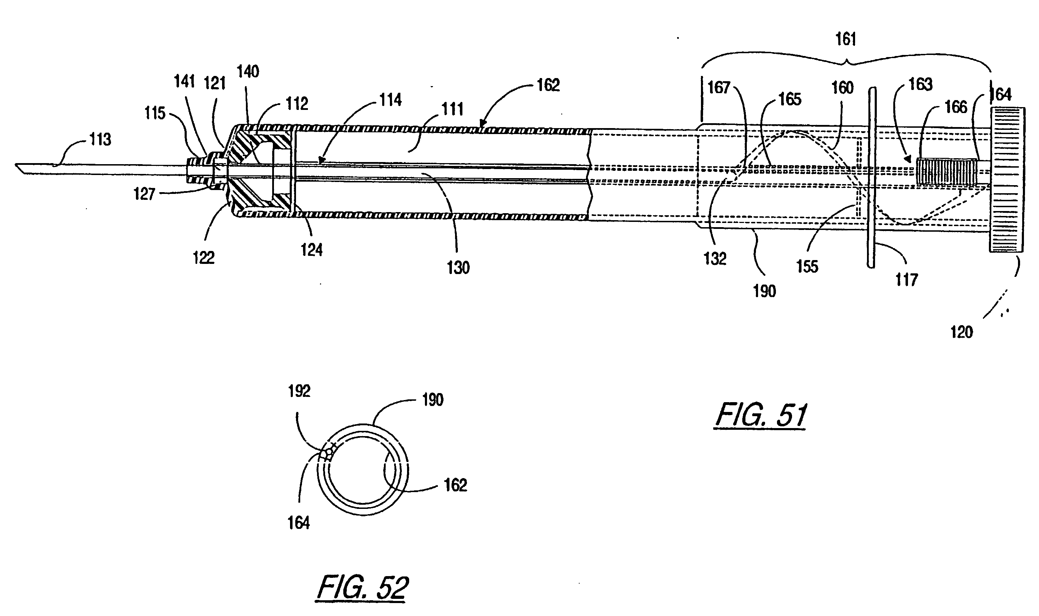

FIG. 51 illustrates a syringe similar to the syringe of FIG. 46 in that the spiral channel 160 extends through the wall of the barrel. In the design of FIG. 51, however, the spiral channel 160 is covered by separate sleeve 190 which is telescoped over, and attached to, the barrel 162. This sleeve 190 also forms the finger flange 117. This construction is particularly desirable when a portion of the spiral channel is located on the distal side of the finger flange 117, because that portion of the barrel is often gripped by the person using the syringe, and the smooth sleeve 190 has a better feel than a barrel surface having a spiral channel extending through the wall.

FIG. 52 is an end view of the proximal end of the barrel 162 and the sleeve 190 with the latch 163 removed. It can be seen that the slot 164 for the latch is formed in both the barrel 162 and the surrounding sleeve 190, and a hole 192 for the latching pin 167 is formed by the mating surfaces of the barrel 160 and the sleeve 190. Half of the hole 192 is formed in the inner surface of the sleeve 190, while the other half of the hole is formed in the outer surface of the barrel 162. When these two members are assembled, the mating surfaces form a circular hole 192 for receiving the latching pin 167.

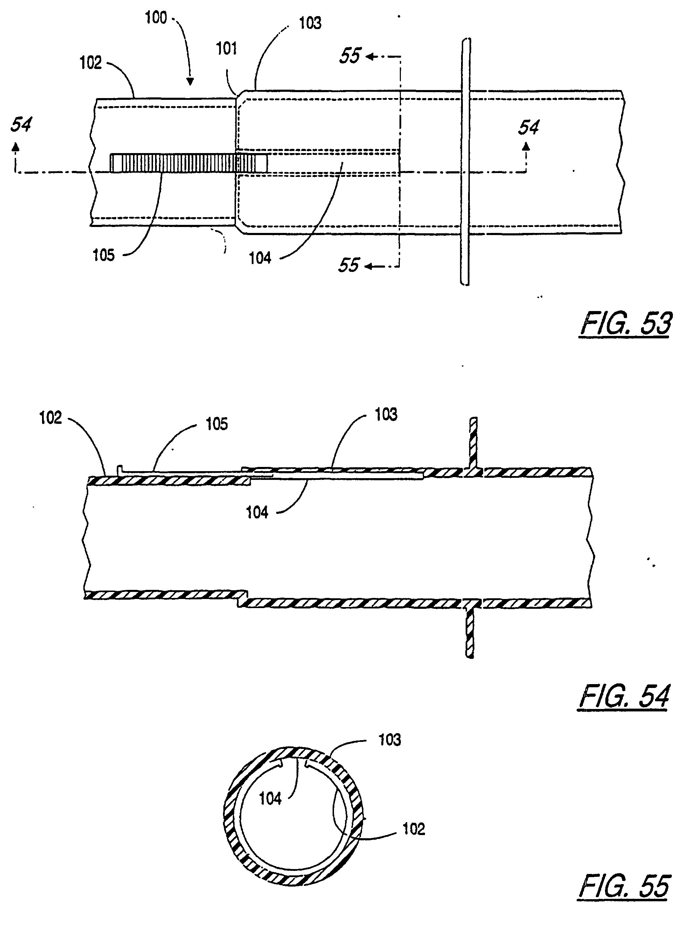

FIGs. 53-55 illustrate another modified barrel and latch. The barrel 100 in this embodiment is stepped at 101 so that both the inside diameter and the outside diameter of the barrel increase in the region containing the spiral channel, which is not shown in FIGs. 52-53. Because of the increase in the inside diameter of the barrel, the plunger will be supported only in the distal portion 102 of the barrel, while in the proximal portion 103 of the barrel a small gap will exist between the outer periphery of the plunger and the inner periphery of the barrel. The plunger will still be coaxially supported within the barrel because a substantial length of the plunger is always engaged within the distal portion 102 of the barrel. A channel 104 is formed in the inside surface of the proximal barrel portion 103 for receiving a latch 105. The sidewalls of the channel 104 guide and stabilize the latch 105 as it is moved back and forth between its advanced (closed) position within the spiral channel and its retracted (open) position outside the spiral channel.

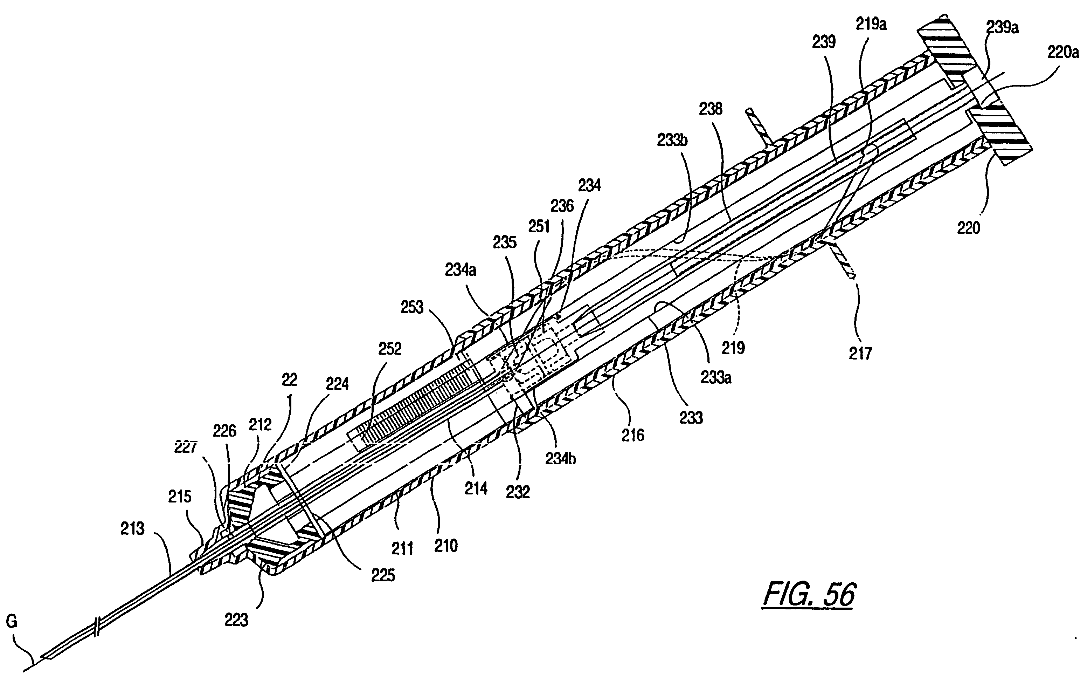

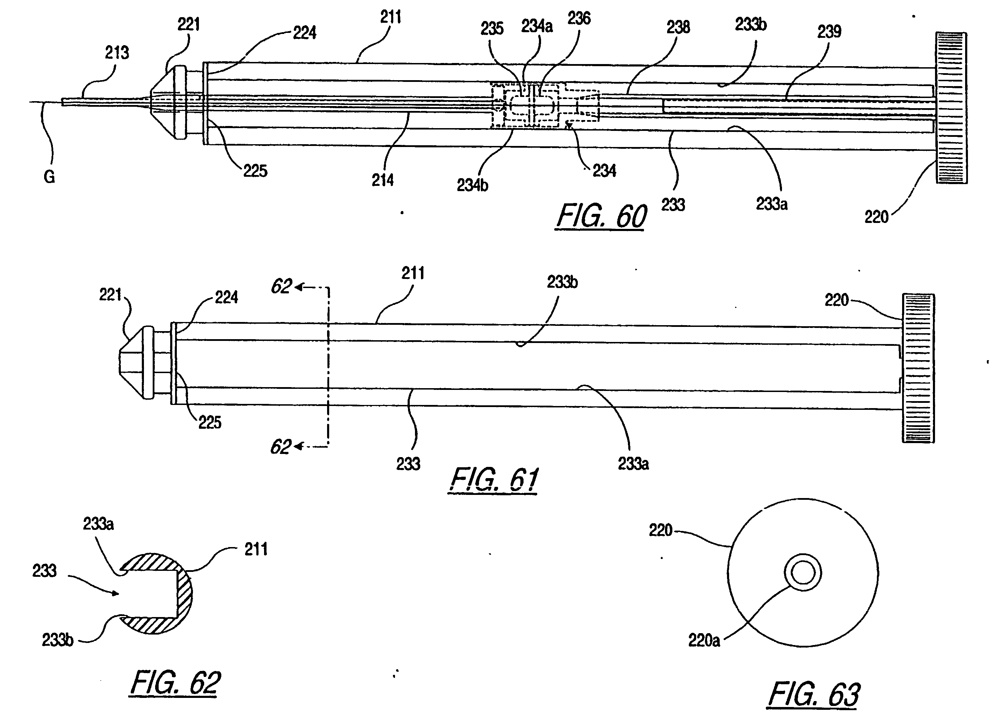

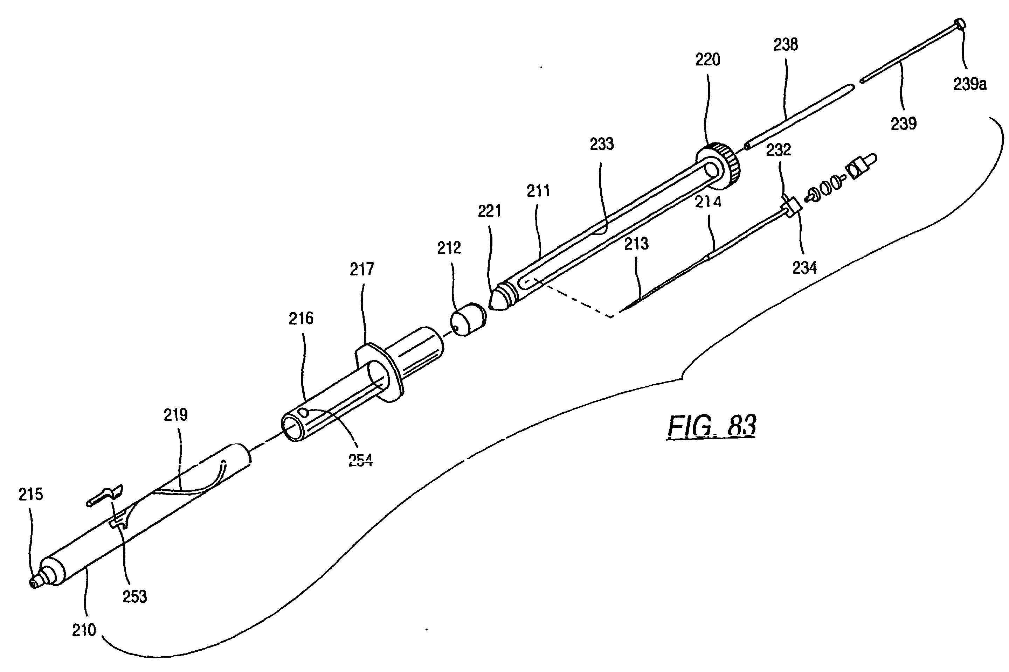

FIGs. 56-73 and 80-83 illustrate a needle-syringe guidewire assembly including a barrel 210, a plunger 211, a hollow plunger cap 212, a hypodermic needle 213, and a needle holder 214. This assembly is used to puncture a patient's vein or artery and then insert a guidewire G through the puncture. The barrel 210 is a hollow cylinder which terminates in a hollow tapered nozzle 215 at the distal end thereof. The interior of the nozzle 215 communicates with the hollow interior of the tubular body portion of the barrel 210. The proximal portion of the barrel is telescoped within, and bonded to, a sleeve 216. An outwardly extending flange 217 near the proximal end of the sleeve 216 facilitates gripping of the syringe with the user's fingers when it is desired to move the plunger 211 relative to the barrel 210.

The proximal end portion of the barrel 210 within the sleeve 216 forms a spiral slot 219 within its wall. As will be described below, this spiral slot 219 provides an internal retraction track for the needle holder 214 and the hypodermic needle 213. The spiral slot 219 extends along a sufficient length to accommodate retraction of the needle holder 214 through a distance that is sufficient to draw the entire length of the needle 213 inside the barrel 210, as described in more detail below. The outer surface of the barrel 210 preferably contains graduations (not shown) indicating the volume level of fluid in the barrel. These graduations take into account the volume of the internal components such as the needle holder 214.

The proximal end of the plunger 211 forms a knob 220 that can be grasped by a user to effect linear or rotary movement of the plunger 211 relative to the barrel 210. The periphery of the knob 220 is serrated to facilitate gripping of the knob for rotary movements of the plunger. The distal end of the plunger 211 forms a head 221 to accommodate the hollow rubber plunger cap 212. The outside diameter of the resilient cap 212 is reduced in the central portion so that the cap engages the inside wall of the barrel 210 only at the pliable margins of the ends of the cap. The diameter of the engaging end portions of the cap 212 is slightly larger than the inside diameter of the barrel 210 so that the cap presses firmly against the inside wall of the barrel to form an air-tight and liquid-tight seal at the cap/barrel interface. The inner margins of the cap 212 make a similar tight contact with the outer surface of the needle holder 214. The distal end 222 of the cap 221 is conical to conform to the conical distal end 223 of the inside surface of the barrel 210 when the plunger 211 is fully advanced within the barrel.

The head 221 of the plunger 211 is configured to fit tightly within the hollow plunger cap 212. With the cap 212 locked onto the head 221 of the plunger, the flat proximal end 224 of the cap abuts the flat surface of a circular disc 225 at the base of the plunger head 221. Due to the air-tight and liquid-tight seal between the plunger cap 212 and the barrel 210, as well as the needle holder 214, advancing movement of the plunger 211 inside the,barrel 210 creates pressure in the interior of the barrel between the plunger cap and the distal end of the barrel. Similarly, retracting movement of the plunger 211 creates a vacuum in that portion of the barrel interior.

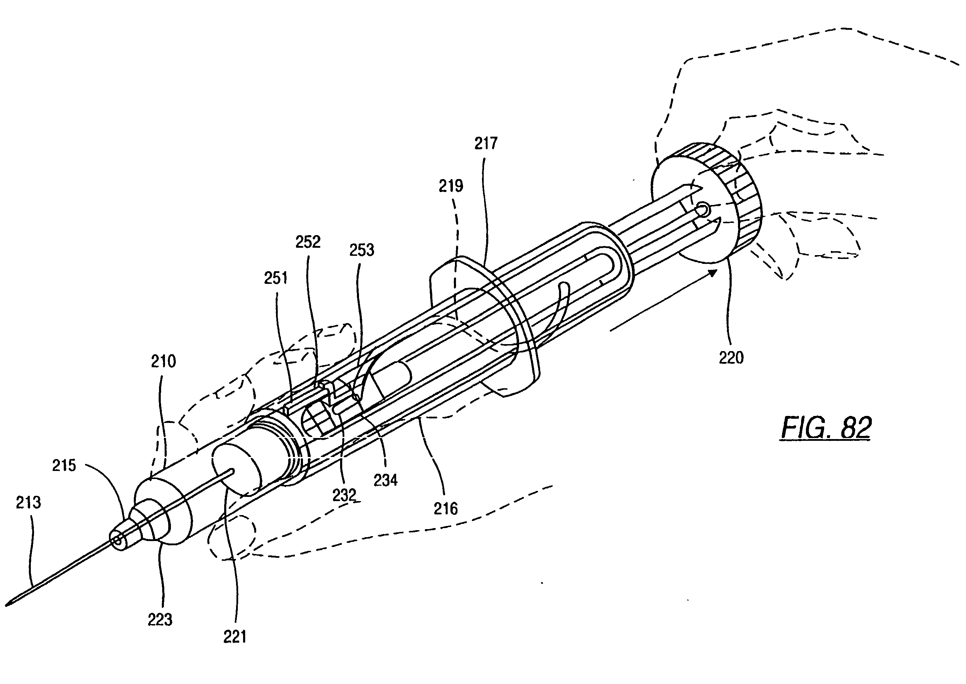

The hypodermic needle 213 projects from the distal end of the elongated needle holder 214, which is detachably interlocked to the barrel 210. Prior to use of the needle-syringe assembly, the needle 213 is covered by a protective cap (not shown) which prevents needle pricks and preserves sterility prior to use. Both the needle 213 and the needle holder 214 are hollow, and the interior of the hollow needle 213 communicates with the interior of the barrel 10 through an aperture 226 in the side walls of the needle 213 and the needle holder 21 (FIG. 82 and FIG. 56). Prior to and during use of the needle-syringe assembly for withdrawal of blood and insertion of the guidewire (hereafter referred to as "normal use"), the aperture 226 is positioned at the base of the barrel nozzle 215 (FIG. 56), within a small cylindrical cavity 227. The aperture 226 permits blood to enter or exit from the barrel 210 via the needle 213 and the needle holder 214.

During normal use of the needle-syringe assembly, the needle holder 214 is locked to the barrel 210, and the plunger 211 and its cap 212 are free to slide longitudinally back and forth along the needle holder. The needle holder 214 includes a lateral arm 232 extending radially across the barrel 210 at the proximal end of the holder 214.

To permit relative sliding movement between the plunger 211 and the needle holder 214 in the longitudinal direction, the needle holder is mounted in a longitudinal channel 233 formed as an integral part of the plunger 211. The proximal end of the needle holder 214 terminates in a small rectangular housing 234 that slides on the bottom wall of the channel 233. The parallel side walls 234a and 234b of the housing slide on the opposed side walls 233a and 233b of the channel 233. The three pairs of engaging walls stabilize the needle holder 214 as it slides longitudinally back and forth within the plunger channel 233, and also constrain the lateral arm 232 of the needle holder against any angular or rotational displacement relative to the plunger 211. That is, the plunger 211 and the needle holder 214 can rotate only in unison with each other, although they are free to move independently of each other in the longitudinal direction. To lock the needle holder 214 to the barrel 210, the outer surface of the distal end portion of the needle holder is tapered to mate with a complementary tapered surface on the inside wall of the barrel nozzle 215.

The locking tapered surfaces are engaged during assembly of the needle syringe, when the plunger 211 and needle holder 214 are inserted into the barrel 210 through the open proximal end of the barrel. The resultant locking luer taper can be released only by the application of simultaneous axial and rotational forces.

The proximal end of the needle holder 214 is also locked to the barrel 210, via the lateral arm 232. This arm 232, which is formed as an integral part of the housing 234, extends radially beyond the plunger and fits into the spiral slot 219 in the barrel 210. The arm 232 can be locked to the barrel 210 at either end of the spiral slot 219 and, when so locked, permits only reciprocal linear movement of the plunger 211, to create vacuum to withdraw blood and pressure to return blood to the patient via the hypodermic needle. When the arm 232 is locked at either end of the slot 219, the plunger 211 cannot be rotated within the barrel 210.

The housing 34 contains a pair of dome valves 235 and 236 which permit the guidewire G to pass through the housing 234 and into the needle holder 214 while preventing blood from flowing proximally from the hollow interior of the needle 213. The two dome valves 235 and 236 face in opposite directions so that one valve prevents blood flow when the plunger is drawing a vacuum in the barrel, and the other valve prevents blood flow when the plunger is producing a positive pressure in the barrel. These valves are sufficiently flexible that finger pressure on the guidewire G is sufficient to cause the guidewire to pass through each valve.

To facilitate entry of the guidewire G into the housing 234 and the valves 235 and 236 therein, the proximal end of the housing 234 may form a tapered entry throat to guide the tip of the guidewire toward the slit in the center of the valve 236. The proximal end of the housing 234 receives, and is attached to, the distal end of a guide tube 238 which telescopes over a smaller guide tube 239. The smaller guide tube 239, which has an inside diameter only slightly larger than the diameter of the guidewire G, extends through a hole in the center of the knob 220 on the proximal end of the plunger 211. A head 239a on the end of the tube 239 nests in a recess 220a in the proximal surface of the knob 220 so that the proximal end of the tube cannot move beyond the knob 220 in a distal direction. This feature limits the distal movement of the tube 239 relative to the plunger 211, while permitting unlimited movement of the tube 239 in a proximal direction relative to the plunger.

The pair of telescoping guide tubes 238 and 239 form a conduit for the guidewire G, leading from the proximal end of the plunger to the entry throat of the housing 234 on the proximal end of the needle holder 214. Consequently, after the hypodermic needle 213 has punctured the patient's vein or artery, the user simply inserts the tip of the guidewire G into the open end of the guide tube 239 and manually advances the guidewire through the two guide tubes 239 and 238, the housing 234 and the two dome valves 235 and 236 therein, and on through the hollow needle 213 into the patient.

When the user desires to retract the hypodermic needle 213 within the barrel-plunger assembly, after the guidewire has been installed and the needle has been withdrawn from the patient and the guidewire, a mechanical latch 250 is manually actuated to unlock the arm 232. This permits rotation of the plunger 211 relative to the barrel 210, which in turn retracts and locks the needle-needle holder assembly within the barrel-plunger assembly. For the needle and needle holder to be moved to the retracted position, the plunger 211 can be in any desired position, e.g., to permit blood or medication to be retained in the syringe.

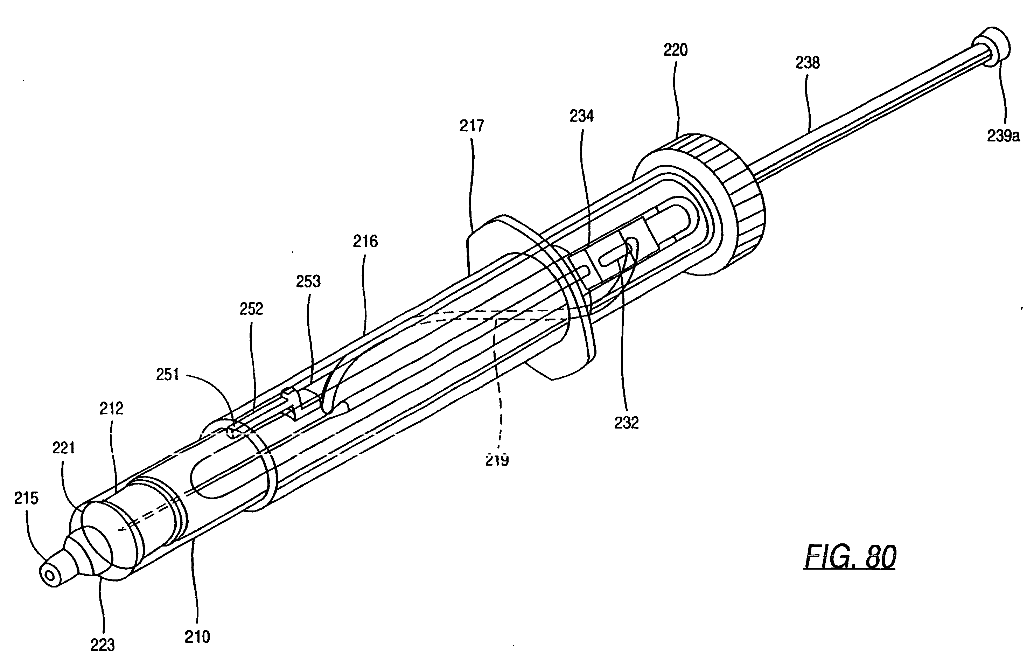

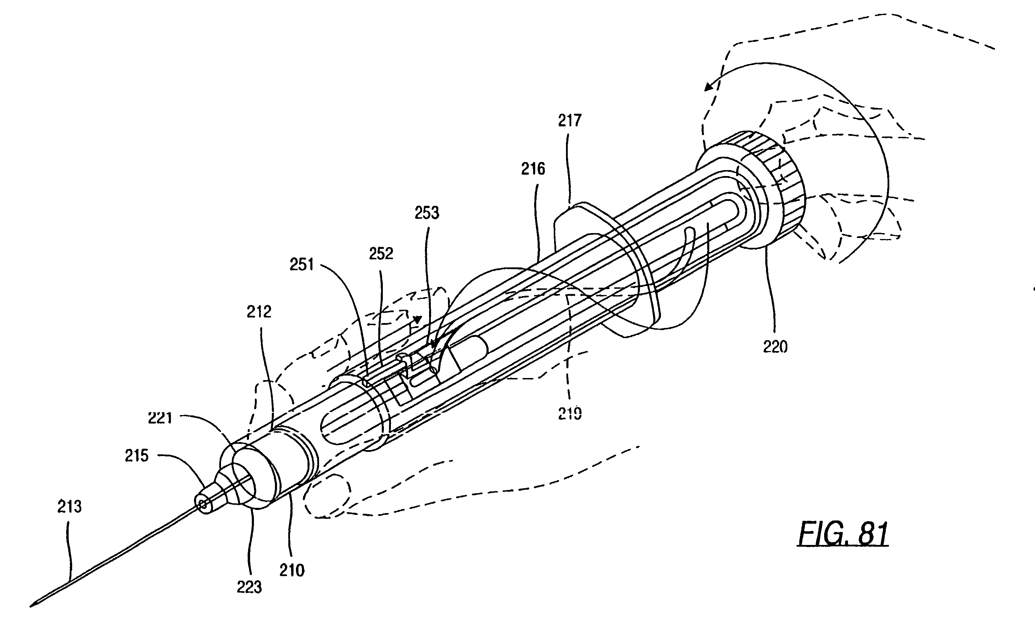

One latch mechanism 250 of FIGs. 80-83 and FIG. 66 includes an arcuate locking plate 251 and an integral handle 252 mounted for sliding movement within a short longitudinal slot 253 in the wall of the barrel 210. The free end of the plate 251 is angled to match the slope of the side walls of the spiral slot 219, and the plate slides back and forth within a slot 254 formed in the sleeve 216 and opening into the slot 219 adjacent the distal end of the slot. The inner and outer radii r1 and r2 of the plate 251 match those of the sleeve 216 (see FIG. 81) to ensure that the locking plate 251 and the handle 252 are offset from each other in the radial direction so that the handle rides on the outer surface of the barrel 210. This outer handle surface is serrated to facilitate movement thereof with the user's finger or thumb.

The latch 250 can be opened or closed by linear movement of the locking plate 251 via the handle 252. During normal use, the needle holder arm 232 is positioned at the distal end of the spiral slot 219 and the locking plate 251 is advanced into the spiral slot 219 to close the slot and retain the arm 232 at the distal end of the slot 219. This locks the needle holder 214 in the normal operative mode in which only linear reciprocal movement of the plunger 211 is permitted. Because the locking plate 251 blocks the spiral slot 219, the needle holder 214 cannot rotate and thus cannot travel along the spiral slot 219 for retraction of the hypodermic needle 213. When it is desired to retract the needle, the latch handle 252 is retracted toward the distal end of the syringe, thereby opening the spiral slot 219 and permitting rotation of the plunger 211 and retraction of the needle holder 214 by movement of the arm 232 along the spiral slot.

It will be appreciated that when the latch 250 is retracted to open the spiral slot 219 and thereby unlock the arm 232, the plunger can be in any desired longitudinal position. That is, the plunger can be fully advanced, fully retracted, or at any intermediate position. This is advantageous because it might be desired to retain a portion of a blood sample withdrawn from a patient within the syringe. To prevent the leakage of any fluid contained within the syringe at the time the needle is retracted, it is preferred to provide a latex seal (not shown) at the end of the nozzle 215.

To ensure retention of the end portion of the arm 232 within the spiral slot 219 during retracting movement of the needle holder 214, the plunger 211, forms a solid cylinder interrupted only by the channel 233. The diameter of this cylinder matches the inside diameter of the barrel 210 so that it tends to maintain the desired circular shape of the inside wall of the barrel. Stresses exerted on the wall of the barrel during use can tend to distort the desired circular configuration of the barrel, and if the distortion becomes large enough, the arm 232 can escape from the spiral slot 219. With the solid cylindrical plunger riding on the inside wall of the sleeve 218, however, such excessive distortion is prevented, and thus retention of the arm 232 within the spiral slot 219 is ensured.

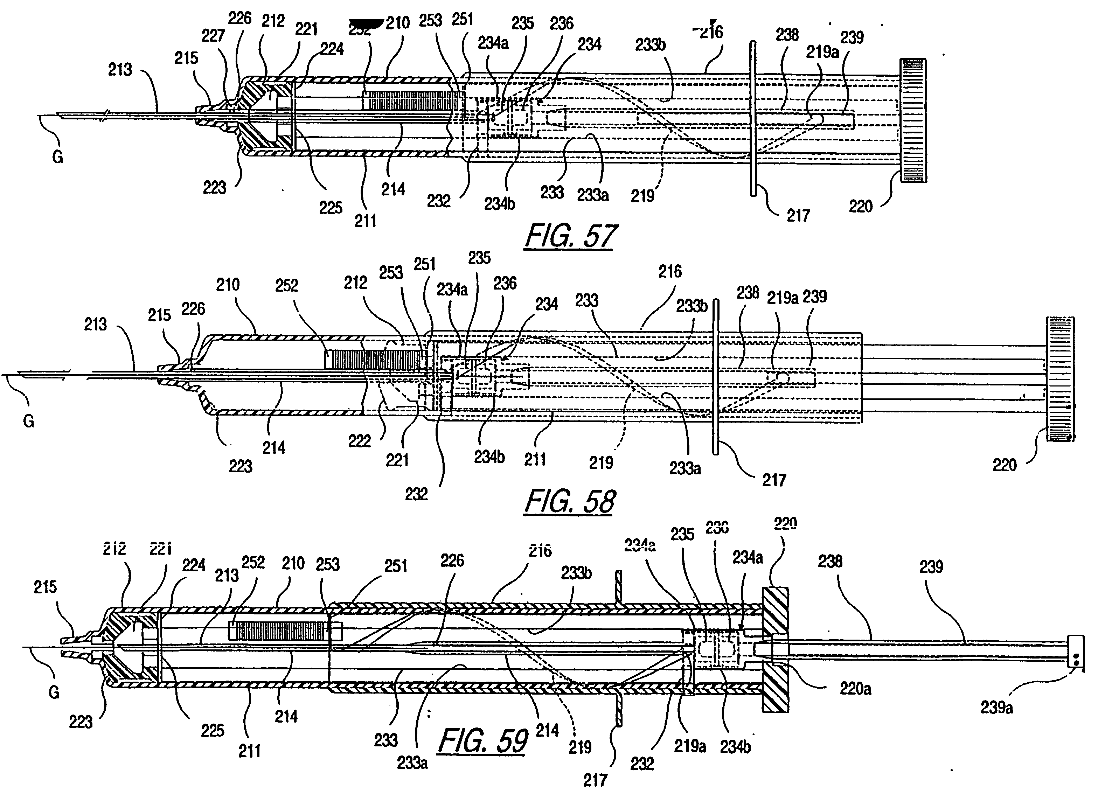

During normal use of the needle-syringe assembly, the barrel 210 and the needle holder 214 are held stationary, and the plunger 211 is free to move relative to both the barrel 210 and the needle holder 214. Advancing movement of the plunger 211 is limited by contact of the plunger cap 212 with the end wall of the barrel 210, as shown in FIGs. 56 and 57. Retracting movement of the plunger 211 is limited by contact of the plunger disc 225 with the arm 232, as shown in FIG. 58. If desired, stop members may be provided on the inside surface of the barrel to engage the disc 225 on the distal side of the latch opening, to further protect against the leakage of fluids through the latch opening in the barrel wall. The needle holder 214 is locked to the barrel 210 by virtue of the taper lock between the distal portion of the needle holder and the barrel nozzle 215, and the locking engagement of the lateral arm 232 in the wall of the barrel. Alternatively, the needle holder can be locked to the nozzle by a threaded connection. The plunger 211 is also free to move longitudinally relative to the needle holder 214, as illustrated in FIG. 58, because the needle holder is not locked to the plunger in that direction. The locking of the lateral arm 232 to the barrel wall prevents rotational movement of the plunger as well as the needle holder, and also prevents the plunger from being accidentally pulled out. As long as the lateral arm 232 of the needle holder is locked to the barrel wall, the needle-syringe assembly is in its normal operating mode.

Following insertion of the guidewire and removal of the needle from the patient, the needle 213 can be retracted into the plunger 211 and the barrel 210, as shown in FIG. 59. This requires axial movement of the needle holder 214 within the barrel 210 toward the proximal end thereof, which in turn requires that the needle holder 214 be unlocked for movement along the spiral slot 219. Thus, to initiate retraction of the needle holder 214, the arm 232 is unlocked by retracting the locking plate 251.

After the latching plate 251 has been retracted, the plunger knob 220 is turned to rotate the plunger 211 clockwise (as viewed from the proximal end) relative to the barrel. As the plunger is rotated, the needle holder 214 rotates in unison with the plunger because the arm 232 is fastened to the housing 234, which in turn is captured between the opposed parallel walls of the channel 232 in the plunger. Rotation of the needle holder 214 relative to the barrel 210 (1) retracts the needle holder within the plunger by the camming action of the wall of the spiral slot 219 acting on the arm 232, and (2) releases the locking luer taper at the distal end of the barrel nozzle 215 due to the resulting compound rotational and longitudinal forces applied to the tapered surfaces. As rotation continues, the arm 232 traverses the entire length of the spiral slot 219, thereby retracting the entire needle holder 214 through a corresponding axial distance within the plunger 211 (see FIG. 59). Of course, the needle 213 is retracted along with the needle holder 214, and thus the needle is retracted completely within the barrel nozzle 210 and the plunger 211, as illustrated in FIG. 59.

As an alternative to the previous embodiment, the spiral slot 219 may be formed in a sleeve fitted inside an enlarged distal end portion of the barrel 210, and attached to the barrel. The spiral slot preferably has a constant rate of curvature along its length. The portion of the barrel 210 that receives the sleeve has a slightly larger diameter than the central portion of the barrel, and the sleeve has the same inside diameter as the central portion of the barrel. Alternatively, a spiral channel can be molded as a part of the inside wall of the end portion of the barrel that has the slightly larger diameter.

At the distal end of the spiral slot 219, the end of the arm 232 snaps into a detent notch 219a formed by the walls of the slot so that the user feels the end of the needle retraction, as a click. Then if the user attempts to turn the plunger knob 220 in the opposite direction, such attempt is met with firm resistance. This is a safety feature to prevent the needle from being returned beyond the end of the barrel nozzle, and to discourage re-use of the needle.

To operate the guidewire-syringe the area of the intended procedure is scrubbed, cleaned and draped, the intended vein or artery is localized by anatomical landmarks and the area is locally anesthetized. After removing the protective cap, the guidewire-syringe is primed with normal saline to remove traces of air. The intended vessel is then punctured and access is confirmed by aspiration of the blood in the syringe. After confirming the vessel entry the aspirated blood is returned to the patient. The straightened guidewire is then fed into the opening at the face plate of the plunger. The guidewire passes through the valve, the needle holder and the hypodermic needle into the patient's vein/artery. Once a required length is introduced, the syringe is withdrawn, leaving the guidewire in place. This leaves the guidewire exiting from the patient's skin in a blood-less environment. The latch handle 252 is then retracted to open the spiral channel 219, and the plunger knob 220 is rotated clockwise until the user feels the arm 232 snap into the detent notch 219a at the proximal end of the spiral slot 219. The spiral slot 219 may alternatively be configured to require counterclockwise, instead of clockwise, rotation of the plunger knob 220. With the needle 213 completely retracted inside the barrel 210, the needle-syringe assembly can be safely discarded in its entirety.

It can be seen from the foregoing description that the needle-syringe-guidewire assembly performs all the conventional functions of guidewire introducers and yet, upon completion of the guidewire installation, the hypodermic needle 213 is concealed within the barrel 210. Another advantage of the needle-syringe assembly is that its design prevents the plunger 211 from slipping out of the barrel 210 during normal use of the assembly.

If desired, the spiral slot 219 may be integrally molded on the inner surface of a proximal end portion of the barrel 210 having an increased wall thickness. Internal molding of the spiral slot is possible when mold cores are rotated while they are pulled from the mold cavity used to form the barrel. A latch mechanism may be mounted in a straight longitudinal slot that opens through the proximal end of the barrel and continues as a straight longitudinal channel on the inside wall of the barrel. The spiral slot preferably extends less than 360 degrees around the circumference of the barrel, and opens through the proximal end of the barrel. This avoids interference between the spiral slot and the latch channel.

The illustrative spiral slot, which extends through the entire thickness of the barrel wall, is preferred because it facilitates molding of the barrel, including the spiral slot, without the use of any special technique. The position of the finger flange and the length of the barrel extension on the proximal side of the flange can be varied as required to retract needles of different lengths.

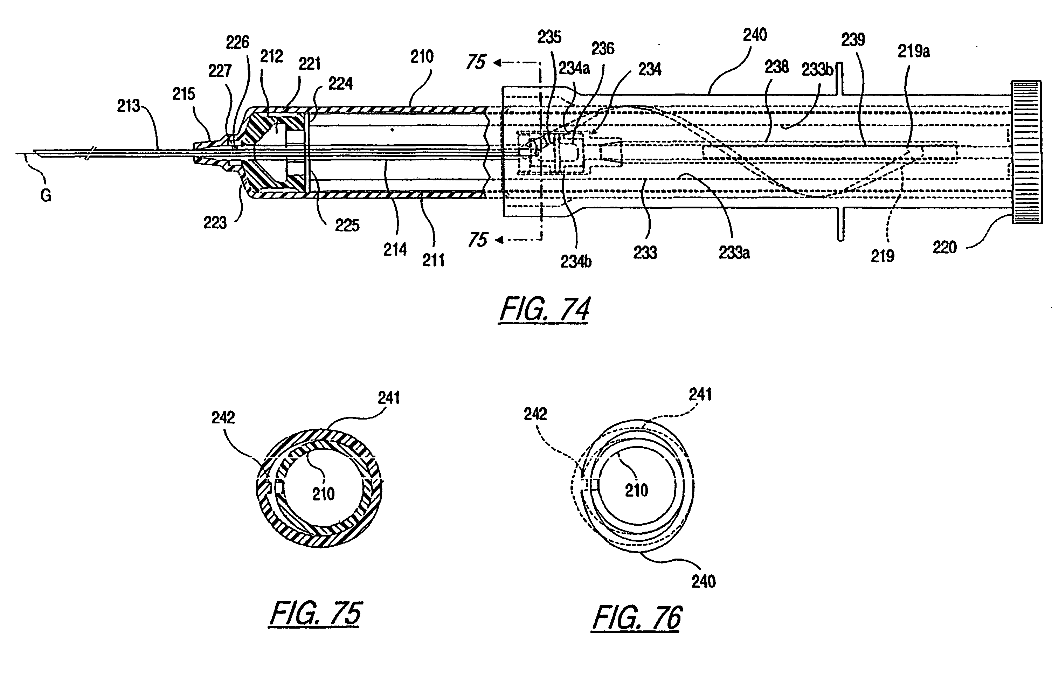

A modified latch arrangement is illustrated in FIGs. 74-76. In this design the spiral slot 219 extends through the wall of the barrel, and a smooth sleeve 240 is telescoped over the proximal end portion of the barrel to cover the slot. The distal end portion 241 of the sleeve 240 is enlarged and has an oval or elliptical transverse cross-section. The minor axis of the ellipse extends over the distal end of the spiral slot 219, and inwardly projecting lug 242 on the inside surface of the sleeve portion 241 of the minor axis extends into the distal end of the slot, on the proximal side of the arm 232. This forms a latch that holds the arm 232 against the distal end of the slot 219. The opposite side of the elliptical end portion 241 is permanently bonded to the outer surface of the barrel 210 by various means such as ultrasonic bonding. When it is desired to release the latch, the user presses on opposite ends of the major axis of the ellipse, thereby converting the elliptical cross-section to a generally circular cross- section. As illustrated by the broken-line illustration in FIG. 76, this deformation causes the lug 242 to be withdrawn from the slot 219, thereby unlatching the arm 232 so that it can be moved along the spiral slot 219. When the deforming pressure is released from the sleeve portion 241, the memory of the resilient plastic material of the sleeve 240 causes the end portion 241 to return to its normal elliptical shape, and the lug 242 re-enters the spiral slot 219.

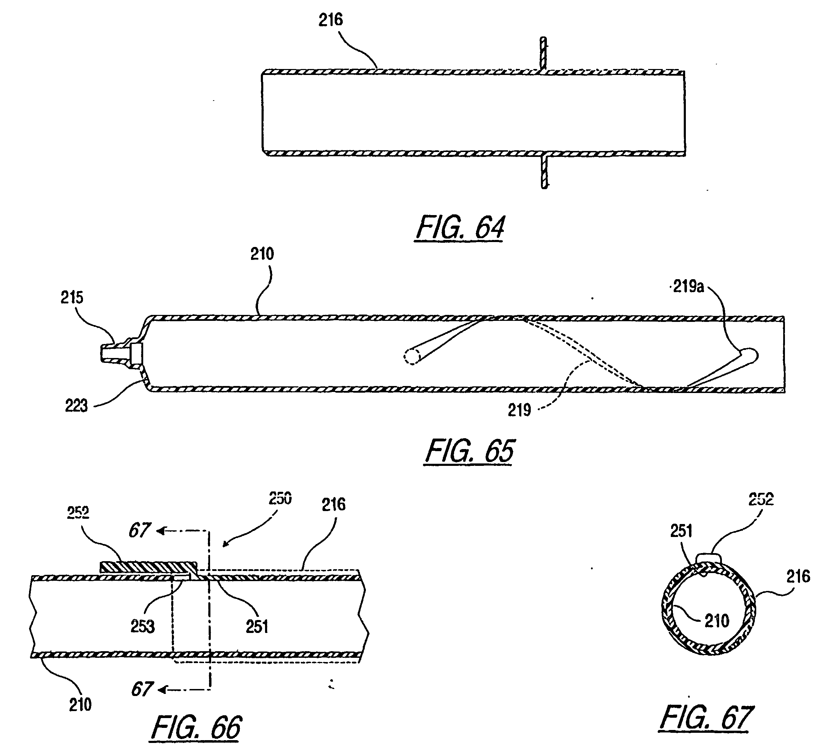

FIGS. 64 and 65 illustrate a modified construction for locking the distal end of the needle holder 214 to the barrel nozzle 215. In this design the plastic insert 242 is modified to form a pair of integral lugs 271 and 272 which thread into a threaded inside surface 273 in the barrel nozzle 215. When the plunger 211 is inserted into the barrel 210 and advanced to the distal end of the barrel, the distal end of the needle holder 214 carried in the plunger enters the nozzle 215. The plunger is automatically rotated by the advancing movement of the arm 232 through the spiral slot 261, and thus the lugs 271,272 are automatically threaded into the nozzle 215. Similarly, when the needle is subsequently retracted, the retracting movement of the arm 232 through the spiral slot 261, and the consequent rotation of the needle holder 214, threads the lugs 271,272 out of the barrel nozzle.

FIGS. 66-68 illustrate a modified plunger and barrel design in which the spiral guide surface is formed by the plunger rather than the barrel. Thus, the proximal end of the plunger 280 includes a hollow cylindrical section 281 adjacent the knob 220, and the wall of this section forms a spiral slot 282. The needle holder arm 232 extends radially outwardly through the slot 282 and into a generally linear channel 283 in the adjacent wall of the barrel 210. Latching holes 284 and 285 are formed in the barrel wall at opposite ends of the channel 283. When the arm 232 is released from the forward latching hole 282, the plunger is turned to move the end of the arm 232 into the channel 283. The arm 232 moves through the hole 282 until it engages the side of the longitudinal section of the channel 283, which then permits longitudinal but not rotational movement of the needle holder as the plunger continues to be turned. Thus, the spiral slot 282 in the plunger acts as a camming surface to urge the arm 232 along the channel 283, thereby retracting the needle 213 into the barrel 210 and ultimately locking it to the barrel margin as described for the first embodiment.

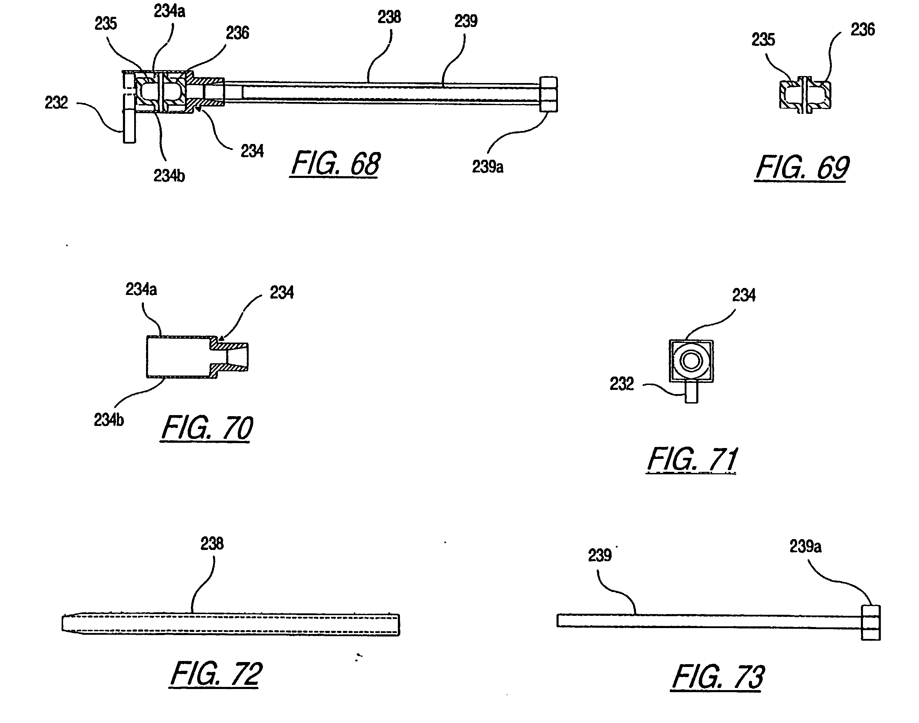

FIG. 69 illustrates a modified design for locking the barrel 262 to the barrel 210. In this case the outside wall of the barrel sleeve 262a forms a resilient lip 290 which snaps into a complementary groove 291 in the inside wall of the barrel body 210a. During sliding movement of the sleeve 262a through the barrel 210a, the lip 290 is bent into an adjoining groove 292 in the sleeve. The interlocking of the lip 290 and the groove 291 prevent relative longitudinal movement between the sleeve and the barrel. A locking tab, of the type described above, can be provided to lock the two members against rotational movement.

FIGs. 71 and 72 show a modified design for the proximal end of the barrel 210. This modified barrel 210b eliminates the need for the collar 250 by providing a circular depression around the latching hole 245. This depression 295 allows a user's finger to engage the end of the lateral arm 232 and to press the arm inwardly to disengage it from the latching hole 245. The plunger is then turned relative to the barrel while the arm 232 is pressed inwardly, to initiate the retracting movement of the needle holder.

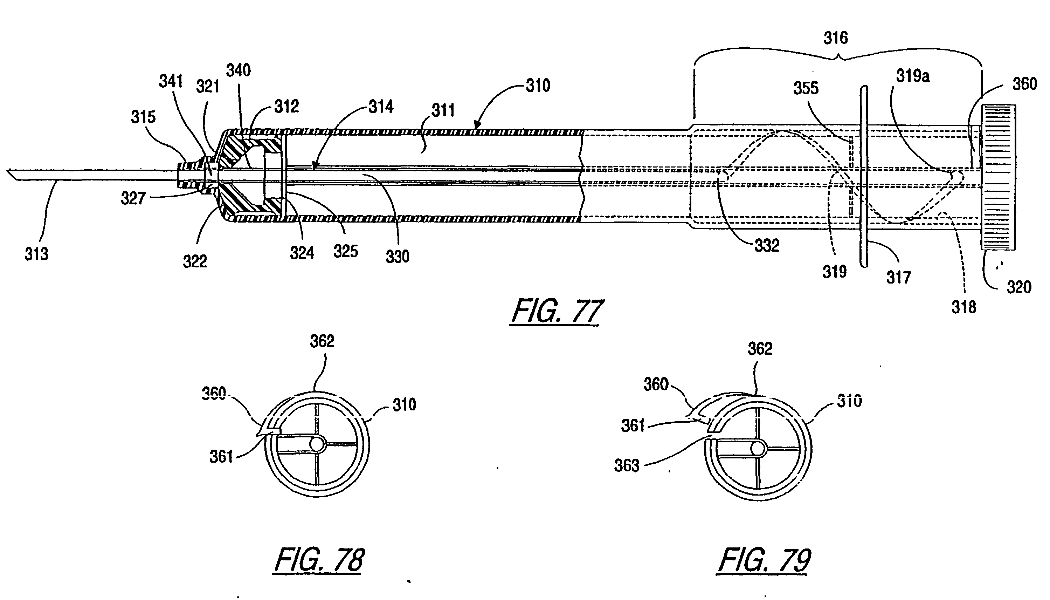

FIGs. 77-79 illustrate another latch arrangement where the spiral slot 119 is formed in a sleeve fitted inside an enlarged end portion 316 of the barrel 310, and attached to the barrel. The spiral slot preferably has a constant rate of curvature along its length. The portion of the barrel 310 that receives the sleeve has a slightly larger diameter than the central portion of the barrel, and the sleeve has the same inside diameter as the central portion of the barrel. The needle-syringe assembly also includes a plunger 311 (with plunger head 321), a hollow plunger cap 312 (with distal end 322), a hypodermic needle 313, a needle holder 314, a hollow tapered nozzle 315, an outwardly extending flange 317, and a knob 320. A flat proximal end 324 of the cap abuts the flat surface of a circular disc 325 at the base of the plunger head 321. The needle holder includes a metal tube 340 and L-shaped metal rod 330. The opposed ends of the needle 313 and the rod 330 are separated from each other, and the intervening space is surrounded by tube 340 to form a cavity 341 through which fluids pass between the hollow interiors of the needle 313 and the barrel 310. To ensure retention of the end portion of the arm 332 within the spiral slot 319 during retracting movement of the needle holder 314, the plunger 311 includes an integral circular retaining plate 355. The diameter of this plate 355 matches the inside diameter of the guide sleeve 318 so that it tends to maintain the desired circular shape of the inside wall of the sleeve 318.

A latching arm 360 and pin 361 are formed integrally with the barrel and are composed of the same resilient plastic material. The arm 360 is attached to barrel 310 by integral hinge 362 which allows for pivoting movement of the arm 360 and pin 361 both toward and away from the barrel 310. The pin 362 fits into a hole 363 which pierces the barrel and opens into the channel in the sleeve of the needle-syringe assembly. Since the pin 361 is only slightly smaller than hole 363, the resulting friction between the side of the hole and the pin 361 retains the pin 361 in the hole 363. The pin 361 is also retained in the hole by the biasing action of arm 360 which, through its resilient composition, urges the pin 361 into the hole 363. Pulling away the pin 361 from the hole 363 allows arm 332 to move along the slot 319 (FIG. 79). At the end of spiral slot 319, the end of arm 332 snaps into a detent notch 319a formed by the walls of the slot so that the user feels the end of the needle retraction as a click. On the other hand, inserting the pin 361 into the hole 363 holds the arm 332 against the end of the slot 319 forming a latch (FIG. 78).

It can be seen from the foregoing description that the needle-syringe assembly performs all the conventional functions of injection syringes and yet, upon completion of injection, the hypodermic needle is concealed within the barrel. The needle-syringe assembly of this invention is also easy to manufacture, cost-effective, and easy to use in the field. The parts can all be made by conventional plastic molding and using readily available metal needle stock. The final assembly is compact because the needle holder is retracted directly into the plunger itself, and thus the plunger need not be fully extended for needle retraction to occur. When discarded following use, the needle-syringe assembly contributes minimally to the bulk of refuse.

## Claims

1. A needle-syringe assembly, comprising:
an elongated, generally cylindrical barrel (10, 110, 162, 210, 310) forming a hollow nozzle (15, 115, 215, 315) located at the distal end of said barrel and opening into the interior of said barrel;
a plunger (11, 111, 211, 311) slidably mounted in said barrel and farming a longitudinal cavity (33, 133, 233), and a needle holder (14, 114, 214, 314) carrying a hollow needle (13, 113, 213, 313) on the distal end thereof, said needle holder being slidably mounted in said longitudinal cavity of said plunger, said needle holder including a lateral arm (32, 132, 232, 332) extending laterally through said plunger cavity to said barrel;
a guide surface (60, 119, 160, 171, 219, 319) extending along a proximal end portion of said barrel for engaging the lateral arm of the needle holder and retracting the needle holder within the barrel in response to relative rotational movement between the barrel and the needle holder,
**characterised in that**
said guide surface (60, 119, 160, 171, 219, 319) is formed by a groove having sidewalls and a bottom wall.

2. The needle-syringe assembly of claim 1 wherein said guide surface (60, 119, 160, 219, 319) is spiral in shape.

3. the needle-syringe assembly of claim 2 wherein said barrel (10, 110, 310) comprises a sleeve (62, 118, 318) disposed inside the proximal end portion of the barrel and fixed to the barrel.

4. The needle-syringe assembly of claim 3 wherein said sidewalls of spiral guide surface (60, 119, 160, 219, 319) are formed by a spiral slot in said sleeve (62, 118, 318).

5. The needle-syringe assembly of claim 2 which includes means for preventing rotation of said needle holder (14, 114, 214, 314) relative to said plunger (11, 111, 211, 311) so that the needle holder and plunger must rotate in unison.

6. The needle-syringe assembly of claim 2 wherein the lengths of the barrel (10, 110, 162, 210, 310) and the needle holder (14, 114, 214, 314) are selected such that the needle (13, 113, 213, 313) attached to the distal end of the needle holder is fully retracted within the barrel when the lateral arm (32, 132, 232, 332) of the needle holder is retracted to the proximal end of the spiral guide surface (60, 119, 160, 219, 319).

7. The needle-syringe assembly of claim 1 or 2 which includes latching means for releasably latching the needle holder (14, 114, 214, 314) to the barrel (10, 110, 162, 210, 310) when the needle holder is in its fully advanced position.

8. The needle-syringe assembly of claim 7 wherein said latching means comprises a latching hole (45) in the wall of the barrel (10, 210) for receiving the outer end of the lateral arm (32, 232) of the needle holder (14, 214) to latch the needle holder to the barrel, and manually actuatable means (53, 242) for moving the lateral arm inwardly to unlatch the needle holder from the barrel.

9. The needle-syringe assembly of claim 8 wherein a portion of the needle holder (14, 214) adjacent the lateral arm (32, 232) is movable laterally by an amount sufficient to release the outer end of the lateral arm from the latching hole (45) in the barrel wall.

10. The needle-syringe assembly of claim 9 wherein the needle-holder cavity (33) in the plunger (11) has a flexible wall (55) to permit lateral disengaging movement of said arm (32).

11. The needle-syringe assembly of claim 8 wherein the manually actuatable means is a collar (50) captured on the outer surface of the barrel and forming an inwardly extending pin (53) registered with the latching hole (45) in the barrel wall, the portion of the collar adjacent the pin being resilient to enable the pin to be advanced into the latching hole to force the lateral arm (32) of the needle holder (14) out of the latching hole.

12. The needle-syringe assembly of claim 2 wherein said longitudinal cavity (33, 133, 233) is a channel formed as an integral part of the plunger (11, 111, 211, 311).

13. The needle-syringe assembly of claim 12 wherein the opposed walls of said channel (33, 133, 233) engage opposite sides of the lateral arm (32, 132, 232, 332) of the needle holder to prevent the needle holder (14, 114, 214, 314) from rotating relative to the plunger (11, 111, 211, 311).

14. The needle-syringe assembly of claim 13 wherein a locking male luer taper (44, 144) is formed by a polymeric sleeve (42, 142) on a proximal extension of the hollow needle (11, 114, 214, 314).

15. The needle-syringe assembly of claim 2 wherein the distal portion of the inside surface of the hollow nozzle (15, 115) on the barrel (10, 110) forms a locking female luer taper (43, 143), and a distal portion of the outside surface of the needle holder (14, 114) forms a locking male luer taper (44, 144).

16. The needle-syringe assembly of claim 2 wherein mating surfaces of the distal portions of the hollow nozzle (15) on the barrel (10) and the needle holder (14) form cooperating threads (71, 72; 74) for locking the needle holder to the barrel during longitudinal movement of the plunger (11) relative to the barrel.

17. The needle-syringe assembly of claim 16 wherein the threads on said needle holder (14) are formed by a polymeric sleeve (70) molded on a proximal extension of the hollow needle (13).

18. The needle-syringe assembly of claim 2 wherein said spiral guide surface includes a locking detent (66, 119a, 219a) at the proximal end thereof, to resist advancing movement of the needle holder (14, 114, 214) after it has been fully retracted.

19. The needle-syringe assembly of claim 7 wherein said latching means comprises a latching hole (45) in the wall of the barrel (10) for receiving the outer end of the lateral arm (32) of the needle holder (14) to latch the needle holder to the barrel, and the outside surface of the barrel forms a depression (95) around said latching hole to permit a finger to engage the end of said lateral arm and move the arm inwardly to disengage the arm from the barrel.

20. The needle-syringe assembly of claim 2 which includes locking means (66, 119a, 219a) for locking the needle holder (14, 114, 214) to the barrel in response to movement of the needle holder to its retracted position.

21. The needle-syringe assembly of claim 20 which includes second locking means (67) for locking the plunger (11) to the needle holder (14) in response to movement of the needle holder to its retracted position.

22. The needle-syringe assembly of claims 1, 2 and 7 comprising:
a hollow needle holder (214),
hollow guide means (238, 239) mounted between the proximal ends of said needle holder and said plunger (211) for guiding a guidewire (G) from the proximal ends of said plunger to the proximal end of said needle holder,
said needle holder including valve means (235, 236) for passing a guidewire therethrough while preventing blood from flowing between the interiors of said needle holder and said hollow guide means.

23. The needle-syringe assembly of claim 22 wherein said valve means comprises a pair of oppositely facing dome valves (235, 236).

24. The needle-syringe assembly of claim 22 wherein said hollow guide means comprises a pair of telescoping tubular members (238, 239) extending between the proximal ends of said needle holder (214) and said plunger (211) to permit relative longitudinal movement between said needle holder and plunger.

25. The needle-syringe assembly of claim 24 wherein the distal end of one of said telescoping members (238) is attached to said needle holder (214), and the proximal end of the other telescoping member (239) is blocked from moving inside the plunger (211).

26. The needle-syringe assembly of claim 24 wherein the proximal end of said plunger (211) forms an opening into the interior of said telescoping tubular members (238, 239) for permitting the insertion of a guidewire (G) into said tubular members.

27. The needle-syringe assembly of claim 24 wherein the interior of said telescoping tubular members (238, 239) is longitudinally aligned with, and in communication with, the interior of said needle holder (214).

28. The needle-syringe assembly of claims 7 or 22 wherein said latching means comprises a locking element (151, 167, 251) slidably mounted on said barrel (110, 162, 210) for reciprocating movement between a locking position within said guide surface (119, 160, 219) and a non-locking position outside said guide surface.

29. The needle-syringe assembly of claim 28 wherein said latching means includes a manually actuatable handle (152, 166, 252) attached to said locking element (151, 167, 253) and exposed on the outer surface of said barrel (110, 162, 210) for effecting sliding movement of said locking element

30. The needle-syringe assembly of claims 7 or 22 wherein said barrel includes an outwardly extending finger flange (117) to facilitate gripping of the barrel, the distal end of said guide surface (171) terminates within said flange, and said needle holder (114) extends radially through the barrel wall to said flange.

31. The needle-syringe assembly of claim 30 wherein said latching means (174) is movable over said flange (117) to capture the portion (132) of said needle holder (114) that extends into said flange.

32. The needle-syringe assembly of claims 7 or 22 wherein said needle holder (14, 114, 214, 314) includes a lateral arm (32, 132, 232, 332) extending laterally through said plunger cavity (33, 133, 233) to said barrel (10, 110, 162, 210, 310) and said latching means is mounted for movement in and out of said guide surface (60, 119, 160, 171, 219, 319) for capturing and releasing said needle holder arm at the distal end of said guide surface.

33. The needle-syringe assembly of claim 32 wherein said latching means is slidably mounted in a channel formed in said barrel and opening into said guide surface (119, 160, 219) at one end of the channel, and opening through the end of said barrel (110, 162, 210) at the other end of the channel.

34. The needle-syringe assembly of claims 7 or 22 wherein said plunger (11, 111, 211, 311) includes contacting means extending traversely across the interior of said barrel (10, 110, 162, 210, 310) and sliding along the interior surface of said barrel that forms said guide suface (60, 119, 160, 219, 319) so as to maintain the desired configuration of that surface and thereby ensure engagement of the needle holder (14, 114, 214, 314) with said guide surface.

35. The needle-syringe assembly of claim 34 wherein said contacting means includes a circular plate (25, 125, 225, 325) formed as an integral part of said plunger (11, 111, 211, 311).

36. The needle-syringe assembly of claim 35 wherein said contacting means also includes a plurality of longitudinal elements formed as integral parts of said plunger (11, 111, 311) and sliding along the interior surface of said barrel (10, 110, 162, 310) to maintain the desired configuration of that surface.

37. The needle-syringe assembly of claims 7 or 22 wherein said guide surface (60, 119, 160, 171, 219, 319) is formed in the wall of said barrel (10, 110, 162, 210, 310) and extends radially through the wall of said barrel.

38. The needle-syringe assembly of claim 37 which includes an outer sleeve (216) telescoped over said barrel (210) and covering at least a distal end portion of said guide surface (219).

39. The needle-syringe assembly of claim 22 wherein said guide surface (60) includes means at the proximal end thereof for resisting advancing movement of said needle holder (14, 114, 214) after it has been fully retracted.

40. The needle-syringe assembly of claim 22 wherein a detent (66, 119a, 219a) is provided at the distal end of said guide surface (60, 119, 160, 219).

41. The needle-syringe assembly of claims 7 or 22 wherein said latching means includes a slidable plate (151, 253) which forms a distal end portion of a side wall of said guide surface (119, 160, 219) when said plate is in a retracted (open) position, and which blocks said guide surface and captures said needle holder (114, 214) at the distal end of said guide surface when said plate is in an advanced (closed) position.

42. The needle-syringe assembly of claims 7 or 22 wherein said latching means includes a longitudinal latching pin (167) slidably mounted in the wall of said barrel (162) for movement between a retracted (open) position outside said guide surface (160) and an advanced (closed) position in which the pin extends across said spiral channel to block the channel and capture said needle holder (114) at the distal end of the guide surface.

43. The needle-syringe assembly of claim 42 wherein said guide surface is a spiral channel (219), which extends radially through the wall of said barrel (210) and which includes a sleeve (216) telescoped over said barrel and covering said guide surface, the mating surfaces of said barrel and said sleeve forming a longitudinal passageway for receiving said latching pin.

44. The needle-syringe assembly of claims 7 or 22 wherein said guide surface is a spiral groove and the portion (116) of said barrel (110) containing said spiral channel (119) has a larger inside diameter than the distal portion of said barrel, and said latching means (150) slides on the outer surface of said distal portion of the barrel and on the inner surface of the barrel portion containing the spiral groove.

45. The needle-syringe assembly of claim 44 wherein the inner surface of the barrel portion (116) containing the spiral groove (119) forms a longitudinal channel (153) for receiving and guiding said latching means (150).

46. The needle-syringe assembly of claim 7 or 32 wherein said latching means is formed as an integral part of said barrel and comprises a latching arm and pin for reciprocating movement between a blocking position within said spiral groove and a non-blocking position outside said spiral groove.

## Patentansprüche

1. Eine Nadelspritzenvorrichtung, umfassend:
ein längliches, im Wesentlichen zylindrisches Gehäuse (10, 110, 162, 210, 310), das eine hohle Nadel (15, 115, 215, 315) formt, die an dem distalen Ende des Gehäuses angeordnet ist und sich in das Innere des Gehäuses öffnet;
einen Kolben (11, 111, 211, 311), der verschiebbar in dem Gehäuse angebracht ist und einen länglichen Hohlraum (33, 133, 233) formt, und einen Nadelhalter (14, 114, 214, 314), der eine hohle Nadel (13, 113, 213, 313) an seinem distalen Ende trägt, wobei der Nadelhalter verschiebbar in dem länglichen Hohlraum des Kolbens angebracht ist, wobei der Nadelhalter einen Seitenarm (32, 132, 232, 332) umfasst, der sich seitwärts durch den Kolbenhohlraum in das Gehäuse erstreckt;
eine Führungsfläche (60, 119, 160, 171, 219, 319), die sich entlang eines proximalen Endbereichs des Gehäuses für ein Ineingriffbringen mit dem Seitenarm des Nadelhalters und Zurückziehen des Nadelhalters in dem Gehäuse aufgrund einer relativen Drehbewegung zwischen dem Gehäuse und dem Nadelhalter erstreckt,
**gekennzeichnet dadurch, dass**
die Führungsfläche (60, 119, 160, 171, 219, 319) durch eine Nut mit Seitenwänden und einer Bodenwand geformt ist.

2. Die Nadelspritzenvorrichtung aus Anspruch 1, worin die Führungsfläche (60, 119, 160, 219, 319) eine Spiralform aufweist.

3. Die Nadelspritzenvorrichtung aus Anspruch 2, worin das Gehäuse (10, 110, 310) eine Hülse (62, 118, 318) umfasst, die innerhalb des proximalen Endbereichs des Gehäuses angeordnet ist und an dem Gehäuse befestigt ist.

4. Die Nadelspritzenvorrichtung aus Anspruch 3, worin die Seitenwände der spiralförmigen Führungsfläche (60, 119, 160, 219, 319) geformt sind durch einen Spiralschlitz in der Hülse (62, 118, 318).

5. Die Nadelspritzenvorrichtung aus Anspruch 2, welche Einrichtungen zum Verhindem einer Drehung des Nadelhalters (14, 114, 214, 314) relativ zu dem Kolben (11, 111, 211, 311) aufweist, so dass der Nadelhalter und der Kolben gemeinsam drehen müssen.

6. Die Nadelspritzenvorrichtung aus Anspruch 2, worin die Länge des Gehäuse (10, 110, 162, 210, 310) und der Nadelhalter (14, 114, 214, 314) so gewählt sind, dass die Nadel (13, 113, 213, 313), die an dem distalen Ende des Nadelhalters angebracht ist, vollständig in dem Gehäuse zurückgezogen ist, wenn der Seitenarm (32, 132, 232, 332) des Nadelhalters zu dem proximalen Ende der spiralförmigen Führungsfläche (60, 119, 160, 219, 319) zurückgezogen ist.

7. Die Nadelspritzenvorrichtung aus Anspruch 1 oder 2, welche eine Klinkeneinrichtung zum lösbaren Verriegeln des Nadelhalters (14, 114, 214, 314) an dem Gehäuse (10, 110, 162, 210, 310) umfasst, wenn der Nadelhalter sich in seiner vollständig ausgefahrenen Position befindet.

8. Die Nadelspritzenvorrichtung aus Anspruch 7, worin die Klinkeneinrichtung ein Verriegelungsloch (45) in der Wand des Gehäuses (10, 210) zum Aufnehmen eines Außenendes des Seitenarms (32, 232) von dem Nadelhalter (14, 214) aufweist, um den Nadelhalter an dem Gehäuse zu verriegeln, und eine manuell betätigbare Einrichtung (53, 242) zum einwärts Bewegen des Seitenarms, um den Nadelhalter von dem Gehäuse zu entriegeln.

9. Die Nadelspitzenvorrichtung aus Anspruch 8, worin ein Bereich des Nadelhalters (14, 214) angrenzend des Seitenarms (32, 232) seitwärts bewegbar ist, um einen Betrag, der ausreicht, das Außenende des Seitenarms von dem Verriegelungsloch (45) in der Gehäusewand zu lösen.

10. Die Nadelspritzenvorrichtung aus Anspruch 9, worin der Nadelhalterhohlraum (33) in dem Kolben (11) eine flexible Wand (55) aufweist, um eine seitwärts gerichtete Entriegelungsbewegung des Arms (32) zu ermöglichen.

11. Die Nadelspritzenvorrichtung aus Anspruch 8, worin die manuell betätigbare Einrichtung eine Muffe (50) ist, die an der Außenfläche des Gehäuses festgehalten ist und einen einwärts gerichteten Stift (53) formt, der in dem Verriegelungsloch (55) in der Gehäusewand eingerastet ist, wobei der Bereich der Muffe angrenzend des Stifts nachgiebig ist, um dem Stift zu ermöglichen, in das Verriegelungsloch vorgeschoben zu werden, um den Seitenarm (32) des Nadelhalters (14) aus dem Verriegelungsloch zu drücken.

12. Die Nadelspritzenvorrichtung aus Anspruch 2, worin der längliche Hohlraum (33, 133, 233) ein Kanal ist, der als ein integraler Bestandteil des Kolbens (11, 111, 211, 311) geformt ist.

13. Die Nadelspritzenvorrichtung aus Anspruch 12, worin die gegenüberliegenden Wände des Kanals (33, 133, 233) gegenüberliegende Seiten des Seitenarms (32, 132, 232, 332) des Nadelhalters berühren, um den Nadelhalter (14, 114, 214, 314) am Drehen relativ zu dem Kolben (11, 111, 211, 311) zu hindem.

14. Die Nadelspritzenvorrichtung aus Anspruch 13, worin ein Arretiersteck-Luerkegel (44, 144) geformt ist durch eine Polymerhülse (42, 142) an einer proximalen Verlängerung der Hohlnadel (11, 114, 214, 314).

15. Die Nadelspritzenvorrichtung aus Anspruch 2, worin der distale Bereich der Innenfläche der Hohlnadel (15, 115) an dem Gehäuse (10, 110) einen Arretieraufnahme-Luerkegel (43, 143) formt und ein distaler Bereich der Außenfläche des Nadelhalters (14, 114) einen Arretiersteck-Luerkegel (44, 144) formt.

16. Die Nadelspritzenvorrichtung aus Anspruch 2, worin passende Oberflächen der distalen Bereiche der Hohinadel (15) an dem Gehäuse (10) und dem Nadelhalter (14) zusammenarbeitende Gewinde (71, 72; 74) zum Arretieren des Nadelhalters an dem Gehäuse während der Längsbewegung des Kolbens (11) relativ zu dem Gehäuse formen.

17. Die Nadelspritzenvorrichtung aus Anspruch 16, worin das Gewinde an dem Nadelhalter (14) durch eine Polymerhülse (70) geformt ist, die an einer proximalen Verlängerung der Hohlnadel (13) angeformt ist.

18. Die Nadelspritzenvorrichtung aus Anspruch 2, worin die spiralförmige Führungsfläche eine Arretierung (66, 119a, 219a) an ihrem proximalen Ende aufweist, um einer Vorwärtsbewegung des Nadelhalters (14, 114, 214) nachdem dieser vollständig zurückgezogen wurde, zu widerstehen.

19. Die Nadelspritzenvorrichtung aus Anspruch 7, worin die Verriegelungseinrichtung ein Verriegelungsloch (45) in der Wand des Gehäuses (10) zum Aufnehmen des Seitenarms (32) des Nadelhalters (14) aufweist, um den Nadelhalter an dem Gehäuse zu verriegeln, und die Außenseite des Gehäuses eine Vertiefung (95) um das Verriegelungsloch formt, um einem Finger zu ermöglichen, das Ende des Seitenarms zu berühren und den Arm einwärts zu bewegen, um den Arm von dem Gehäuse außer Eingriff zu bringen.

20. Die Nadelspritzenvorrichtung aus Anspruch 2, welche eine Arretiereinrichtung (66, 119a, 219a) zum Arretieren des Nadelhalters (14, 114, 214) an dem Gehäuse aufgrund der Bewegung des Nadelhalters in seine zurückgezogene Position aufweist.

21. Die Nadelspritzenvorrichtung aus Anspruch 20, welche eine zweite Arretiereinrichtung (67) zum Arretieren des Kolbens (11) an dem Nadelhalter (14) aufgrund der Bewegung des Nadelhalters in seine zurückgezogene Position aufweist.

22. Die Nadelspritzenvorrichtung aus Anspruch 1, 2 und 7 mit:
einem Hohlnadelhalter (214),
einer hohlen Führungseinrichtung (238, 239), die zwischen den proximalen Enden des Nadelhalters und des Kolbens (211) zum Führen eines Führungsdrahts (G) von dem proximalen Ende des Kolbens zu dem proximalen Ende des Nadelhalters angebracht ist,
wobei der Nadelhalter eine Ventileinrichtung (235, 236) zum Durchführen eines Führungsdrahts umfasst, während Blut am Fließen zwischen das Innere des Nadelhalters und der hohlen Führungseinrichtung gehindert ist.

23. Die Nadelspritzenvorrichtung aus Anspruch 22, worin die Ventileinrichtung ein Paar von sich gegenüberliegenden domförmigen Ventilen (235, 236) aufweist.

24. Die Nadelspritzenvorrichtung aus Anspruch 22, worin die hohle Führungseinrichtung ein Paar von ineinander geschobenen rohrförmigen Elementen (238, 239) aufweist, die sich zwischen den proximalen Enden des Nadelhalters (214) und des Kolbens (211) erstrecken, um eine relative Längsbewegung zwischen dem Nadelhalter und dem Kolben zu ermöglichen.

25. Die Nadelspritzenvorrichtung aus Anspruch 24, worin das distale Ende eines der ineinandergeschobenen Elemente (238) an dem Nadelhalter (214) angebracht ist und das proximale Ende des anderen ineinandergeschobenen Endes (239) am Bewegen im Innern des Kolbens (211) blockiert ist.

26. Die Nadelspritzenvorrichtung aus Anspruch 24, worin das proximale Ende des Kolbens (211) eine Öffnung im Innern der ineinandergeschobenen rohrförmigen Elemente (238, 239) zum Ermöglichen des Einführens eines Führungsdrahts (G) in die rohrförmigen Elemente formt.

27. Die Nadelspritzenvorrichtung aus Anspruch 24, worin das Innere der ineinandergeschobenen rohrförmigen Elemente (238, 239) mit dem Innern des Nadelhalters (214) längs ausgerichtet ist und mit diesem verbunden ist.

28. Die Nadelspritzenvorrichtung aus Anspruch 7 oder 22, worin die Verriegelungseinrichtung ein Arretierelement (151, 167, 251) umfasst, das verschiebbar an dem Gehäuse (110, 162, 210) für eine Hin- und Herbewegung zwischen einer Arretierposition in der Führungsfläche (119, 160, 219) und einer entriegelten Position außerhalb der Führungsfläche angebracht ist.

29. Die Nadelspritzenvorrichtung aus Anspruch 28, worin die Verriegelungseinrichtung eine manuell betätigbare Handhabe (152, 166, 252) aufweist, die an dem Arretierelement (151, 167, 253) angebracht ist, und an der Außenfläche des Gehäuses (110, 162, 210) zum Ausüben der Verschiebebewegung des Arretierelements vorsteht.

30. Die Nadelspritzenvorrichtung aus Anspruch 7 oder 22, worin das Gehäuse einen auswärts gerichteten Fingerflansch (117) aufweist, um das Ergreifen des Gehäuses zu unterstützen, wobei das distale Ende der Führungsfläche (171) in dem Flansch endet und der Nadelhalter (114) sich radial durch die Gehäusewand zu dem Flansch erstreckt.

31. Die Nadelspritzenvorrichtung aus Anspruch 30, worin die Verriegelungseinrichtung (174) über den Flansch (117) bewegbar ist, um den Bereich (132) des Nadelhalters (114), der sich in den Flansch erstreckt, festzuhalten.

32. Die Nadelspritzenvorrichtung aus Anspruch 7 oder 22, worin der Nadelhalter (14, 114, 214, 314) einen Seitenarm (32, 132, 232, 332) umfasst, der sich seitwärts durch einen Kolbenhohlraum (33, 133, 233) zu dem Gehäuse (10, 110, 162, 210, 310) erstreckt und die Verriegelungseinrichtung für eine Bewegung in die und aus der Führungsfläche (60, 119, 160, 171, 219, 319) zum Festhalten und Loslassen des Nadelhalterarms an dem distalen Ende der Führungsfläche angebracht ist.

33. Die Nadelspritzenvorrichtung aus Anspruch 32, worin die Verriegelungseinrichtung verschiebbar in einem Kanal angebracht ist, der in dem Gehäuse geformt ist und sich in die Führungsfläche (119, 160, 219) an einem Ende des Kanals hin öffnet, und sich durch das Ende des Gehäuses (110, 162, 210) an dem anderen Ende des Kanals hin öffnet.

34. Die Nadelspritzenvorrichtung aus Anspruch 7 oder 22, worin der Kolben (11, 111, 211, 311) eine Berührungseinrichtung aufweist, die sich quer über das Innere des Gehäuse (10, 110, 162, 210, 310) erstreckt und sich entlang der Innenfläche des Gehäuses verschiebt, die die Führungsfläche (60, 119, 160, 219, 319) formt, so dass die gewünschte Konfiguration dieser Oberfläche erhalten ist und dabei ein Eingriff des Nadelhalters (14, 114, 214, 314) mit der Führungsfläche sichergestellt ist.

35. Die Nadelspritzenvorrichtung aus Anspruch 34, worin die Kontakteinrichtung eine Kreisplatte (25, 125, 225, 325) umfasst, die als integraler Bestandteil des Kolbens (11, 111, 211, 311) ausgeformt ist.

36. Die Nadelspritzenvorrichtung aus Anspruch 35, worin die Kontakteinrichtung ebenso mehrere Längselemente aufweist, die als integrale Bestandteile des Kolbens (11, 111, 311) geformt sind und sich entlang der Innenfläche des Gehäuses (10, 110, 162, 310) verschieben, um die gewünschte Konfiguration der Fläche zu erhalten.

37. Die Nadelspritzenvorrichtung aus Anspruch 7 oder 22, worin die Führungsfläche (60, 119, 160, 171, 219, 319) in der Wand des Gehäuses (10, 110, 162, 210, 310) geformt ist und sich radial durch die Wand des Gehäuses erstreckt.

38. Die Nadelspritzenvorrichtung aus Anspruch 37, welche eine Außenhülse (216) aufweist, die über das Gehäuse (210) geschoben ist und zumindest einen distalen Endbereich der Führungsfläche (219) abdeckt.

39. Die Nadelspritzenvorrichtung aus Anspruch 22, worin die Führungsfläche (60) Einrichtungen an ihrem proximalen Ende zum Widerstehen einer Vorwärtsbewegung des Nadelhalters (14, 114, 214) nachdem diese vollständig zurückgezogen ist, umfasst.

40. Die Nadelspritzenvorrichtung aus Anspruch 22, worin eine Verrastung (66, 119a, 219a) an dem distalen Ende der Führungsfläche (60, 119, 160, 219) bereit gestellt ist.

41. Die Nadelspritzenvorrichtung aus Anspruch 7 oder 22, worin die Verriegelungseinrichtung eine Verschiebeplatte (151, 253) umfasst, die einen distalen Endbereich einer Seitenwand der Führungsfläche (119, 160, 219) formt, wenn die Seitenplatte sich in ihrer zurückgezogenen (offenen) Position befindet und welche die Führungsfläche blockiert und den Nadelhalter (114, 214) an dem distalen Ende festhält, wenn sich die Platte in einer vorgeschobenen (geschlossenen) Position befindet.

42. Die Nadelspritzenvorrichtung aus Anspruch 7 oder 22, worin die Verriegelungseinrichtung einen länglichen Verriegelungsstift (167) aufweist, der verschiebbar in der Wand des Gehäuses (162) zum Bewegen zwischen einer zurückgezogenen (offenen) Position außerhalb der Führungsfläche (160) und einer vorgeschobenen (geschlossenen) Position, in welcher der Stift sich über den spiralförmigen Kanal erstreckt, um den Kanal zu blockieren und den Nadelhalter (114) an dem distalen Ende der Führungsfläche festzuhalten, angebracht ist.

43. Die Nadelspritzenvorrichtung aus Anspruch 42, worin die Führungsfläche ein spiralförmiger Kanal (219) ist, welcher sich radial durch die Wand des Gehäuses (210) erstreckt und welcher eine Hülse (216) umfasst, die über das Gehäuse geschoben ist und die Führungsfläche abdeckt, wobei zueinander passende Flächen des Gehäuses und der Hülse einen länglichen Durchgang zum Aufnehmen des Verriegelungsstifts formen.

44. Die Nadelspritzenvorrichtung aus Anspruch 7 oder 22, worin die Führungsfläche eine spiralförmige Nut ist und der Bereich (116) des Gehäuses (110), der den spiralförmigen Kanal (119) umfasst, einen größeren Innendurchmesser aufweist, als der distale Bereich des Gehäuses, und wobei die Verriegelungseinrichtung (150) entlang der Außenfläche des distalen Bereichs des Gehäuses und an der Innenfläche des Gehäusebereichs, der die spiralförmige Nut umfasst, gleitet.

45. Die Nadelspritzenvorrichtung aus Anspruch 44, worin die Innenfläche des Gehäusebereichs (116), der die spiralförmige Nut (119) enthält, einen Längskanal (153) zum Aufnehmen und Führen der Verriegelungseinrichtung (150) umfasst.

46. Die Nadelspritzenvorrichtung aus Anspruch 7 oder 32, worin die Verriegelungseinrichtung als integraler Bestandteil des Gehäuses geformt ist und einen Verriegelungsarm und einen Stift zum Hin- und Herbewegen zwischen einer Blockierposition in der spiralförmigen Nut und einer Nichtblockierposition außerhalb der spiralförmigen Nut umfasst.

## Revendications

1. Ensemble seringue-aiguille, comprenant :
un corps généralement cylindrique, allongé (10, 110, 162, 210, 310) formant une buse creuse (15, 115, 215, 315) située à une extrémité distale dudit corps et s'ouvrant dans l'intérieur dudit corps ;
un plongeur (11, 111, 211, 311) monté en glissement dans ledit corps et formant une cavité longitudinale (33, 133, 233), et un porte-aiguille (14, 114, 214, 314) supportant une aiguille creuse (13, 113, 213, 313) sur son extrémité distale, ledit porte-aiguille étant monté en glissement dans ladite cavité longitudinale dudit plongeur, ledit porte-aiguille comprenant un bras latéral (32, 132, 232, 332) s'étendant latéralement à travers ladite cavité de plongeur jusqu'au dit corps ;
une surface de guidage (60, 119, 160, 171, 219, 319) s'étendant le long d'une portion d'extrémité proximale dudit corps pour engager le bras latéral du porte-aiguille et rétracter le porte-aiguille au sein du corps en réponse à un mouvement de rotation relatif entre le corps et le porte-aiguille,
**caractérisé en ce que**
ladite surface de guidage (60, 119, 160, 171, 219, 319) est formée par une rainure comportant des parois latérales et une paroi inférieure.

2. Ensemble seringue-aiguille selon la revendication 1, dans lequel ladite surface de guidage (60, 119, 160, 219, 319) est en forme de spirale.

3. Ensemble seringue-aiguille selon la revendication 2, dans lequel ledit corps (10, 110, 310) comprend un manchon (62, 118, 318) disposé à l'intérieur de la portion d'extrémité proximale du corps et fixé au corps.

4. Ensemble seringue-aiguille selon la revendication 3, dans lequel lesdites parois latérales de la surface de guidage en spirale (60, 119, 160, 219, 319) sont formées par une fente en spirale dans ledit manchon (62, 118, 318).

5. Ensemble seringue-aiguille selon la revendication 2, qui comprend un moyen pour empêcher une rotation dudit porte-aiguille (14, 114, 214, 314) relativement audit plongeur (11, 111, 211, 311) de manière que le porte-aiguille et le plongeur doivent tourner à l'unisson.

6. Ensemble seringue-aiguille selon la revendication 2, dans lequel les longueurs du corps (10, 110, 162, 210, 310) et du porte-aiguille (14, 114, 214, 314) sont sélectionnées de manière que l'aiguille (13, 113, 213, 313) fixée à l'extrémité distale du porte-aiguille soit complètement rétractée au sein du corps lorsque le bras latéral (32, 132, 232, 332) du porte-aiguille est rétracté jusqu'à l'extrémité proximale de la surface de guidage en spirale (60, 119, 160, 219, 319).

7. Ensemble seringue-aiguille selon la revendication 1 ou 2, qui comprend un moyen de verrouillage pour verrouiller, avec possibilité de libération, le porte-aiguille (14, 114, 214, 314) au corps (10, 110, 162, 210, 310) lorsque le porte-aiguille est dans sa position complètement déployée.

8. Ensemble seringue-aiguille selon la revendication 7, dans lequel ledit moyen de verrouillage comprend un orifice de verrouillage (45) dans la paroi du corps (10, 210) pour recevoir l'extrémité externe du bras latéral (32, 232) du porte-aiguille (14, 214) afin de verrouiller le porte-aiguille au corps, et un moyen actionnable manuellement (53, 242) pour déplacer le bras latéral vers l'intérieur afin de déverrouiller le porte-aiguille du corps.

9. Ensemble seringue-aiguille selon la revendication 7, dans lequel une portion du porte-aiguille (14, 214) adjacente au bras latéral (32, 232) est mobile latéralement sur une distance suffisante pour libérer l'extrémité externe du bras latéral de l'orifice de verrouillage (45) dans la paroi du corps.

10. Ensemble seringue-aiguille selon la revendication 9, dans lequel la cavité de porte-aiguille (33) dans le plongeur (11) comporte une paroi flexible (55) pour permettre un mouvement de dégagement latéral dudit bras (32).

11. Ensemble seringue-aiguille selon la revendication 8, dans lequel le moyen actionnable manuellement est un collier (50) capturé sur la surface externe du corps et formant une tige s'étendant vers l'intérieur (53) coïncidant avec l'orifice de verrouillage (45) dans la paroi du corps, la portion du collier adjacente à la tige étant élastique pour permettre à la tige d'être avancée dans l'orifice de verrouillage afin d'obliger le bras latéral (32) du porte-aiguille (14) à sortir de l'orifice de verrouillage.

12. Ensemble seringue-aiguille selon la revendication 2, dans lequel ladite cavité longitudinale (33, 133, 233) est un canal formé d'un seul tenant avec le plongeur (11, 111, 211, 311).

13. Ensemble seringue-aiguille selon la revendication 12, dans lequel les parois opposées dudit canal (33, 133, 233) engagent des côtés opposés du bras latéral (32, 132, 232, 332) du porte-aiguille pour empêcher le porte-aiguille (14, 114, 214, 314) de tourner relativement au plongeur (11, 111, 211, 311).

14. Ensemble seringue-aiguille selon la revendication 13, dans lequel un cône de Luer mâle de verrouillage (44, 144) est formé par un manchon polymère (42, 142) sur une extension proximale de l'aiguille creuse (11, 114, 214, 314).

15. Ensemble seringue-aiguille selon la revendication 2, dans lequel la portion distale de la surface interne de la buse creuse (15, 115) sur le corps (10, 110) forme un cône de Luer femelle de verrouillage (43, 143), et une portion distale de la surface externe du porte-aiguille (14, 114) forme un cône de Luer mâle de verrouillage (44, 144).

16. Ensemble seringue-aiguille selon la revendication 2, dans lequel des surfaces d'ajustement des portions distales de la buse creuse (15) sur le corps (10) et le porte-aiguille (14) forment des filets qui coopèrent (71, 72 ; 74) pour verrouiller le porte-aiguille au corps durant le mouvement longitudinal du plongeur (11) relativement au corps.

17. Ensemble seringue-aiguille selon la revendication 16, dans lequel les filets sur ledit porte-aiguille (14) sont formés par un manchon polymère (70) moulé sur une extension proximale de l'aiguille creuse (13).

18. Ensemble seringue-aiguille selon la revendication 2, dans lequel ladite surface de guidage en spirale comprend un cliquet de verrouillage (66, 119a, 219a) à son extrémité proximale, pour résister au mouvement d'avancée du porte-aiguille (14, 114, 214) une fois qu'il est complètement rétracté.

19. Ensemble seringue-aiguille selon la revendication 7, dans lequel ledit moyen de verrouillage comprend un orifice de verrouillage (45) dans la paroi du corps (10) pour recevoir l'extrémité externe du bras latéral (32) du porte-aiguille (14) afin de verrouiller le porte-aiguille au corps, et la surface externe du corps forme un creux (95) autour dudit orifice de verrouillage (45) pour permettre à un doigt d'engager l'extrémité dudit bras latéral et de déplacer le bras vers l'intérieur afin de dégager le bras du corps.

20. Ensemble seringue-aiguille selon la revendication 2, qui comprend un moyen de verrouillage (66, 119a, 219a) pour verrouiller le porte-aiguille (14, 114, 214) au corps en réponse au déplacement du porte-aiguille jusqu'à sa position rétractée.

21. Ensemble seringue-aiguille selon la revendication 20, qui comprend un deuxième moyen de verrouillage (67) pour verrouiller le plongeur (11) au porte-aiguille (14) en réponse au déplacement du porte-aiguille jusqu'à sa position rétractée.

22. Ensemble seringue-aiguille selon les revendications 1, 2 et 7, comprenant :
un porte-aiguille creux (214),
un moyen de guidage creux (238, 239) monté entre les extrémités proximales dudit porte-aiguille et dudit plongeur (211) pour guider un fil-guide (G) des extrémités proximales dudit plongeur à l'extrémité proximale dudit porte-aiguille,
ledit porte-aiguille comprenant un moyen de valve (235, 236) pour passer un fil-guide au travers tout en empêchant le sang de s'écouler entre les parties intérieures dudit porte-aiguille et dudit moyen de guidage creux.

23. Ensemble seringue-aiguille selon la revendication 22, dans lequel ledit moyen de valve comprend une paire de valves en forme de coupole, opposées, se faisant face (235, 236).

24. Ensemble seringue-aiguille selon la revendication 22, dans lequel ledit moyen de guidage creux comprend une paire d'éléments tubulaires télescopiques (238, 239) s'étendant entre les extrémités proximales dudit porte-aiguille (214) et dudit plongeur (211) pour permettre un mouvement longitudinal relatif entre ledit porte-aiguille et ledit plongeur.

25. Ensemble seringue-aiguille selon la revendication 24, dans lequel l'extrémité distale d'un desdits éléments télescopiques (238) est fixée audit porte-aiguille (214), et l'extrémité proximale de l'autre élément télescopique (239) est empêchée de se mouvoir à l'intérieur du plongeur (211).

26. Ensemble seringue-aiguille selon la revendication 24, dans lequel l'extrémité proximale dudit plongeur (211) forme une ouverture dans l'intérieur desdits éléments tubulaires télescopiques (238, 239) pour permettre l'insertion d'un fil-guide (G) dans lesdits éléments tubulaires.

27. Ensemble seringue-aiguille selon la revendication 24, dans lequel l'intérieur desdits éléments tubulaires télescopiques (238, 239) est aligné longitudinalement avec, et est en communication avec, l'intérieur dudit porte-aiguille (214).

28. Ensemble seringue-aiguille selon la revendication 7 ou 22, dans lequel ledit moyen de verrouillage comprend un élément de verrouillage (151, 167, 251) monté en glissement sur ledit corps (110, 162, 210) pour un mouvement de va-et-vient entre une position de verrouillage au sein de ladite surface de guidage (119, 160, 219) et une position de non-verrouillage à l'extérieur de ladite surface de guidage.

29. Ensemble seringue-aiguille selon la revendication 28, dans lequel ledit moyen de verrouillage comprend une manette actionnable manuellement (152, 166, 252) fixée audit élément de verrouillage (151, 167, 253) et exposée sur la surface externe dudit corps (110, 162, 210) pour effectuer un mouvement glissant dudit élément de verrouillage.

30. Ensemble seringue-aiguille selon la revendication 7 ou 22, dans lequel ledit corps comprend un rebord à doigts s'étendant vers l'extérieur (117) pour faciliter la préhension du corps, l'extrémité distale de ladite surface de guidage (171) se termine au sein dudit rebord, et ledit porte-aiguille (114) s'étend radialement à travers la paroi de corps jusqu'audit rebord.

31. Ensemble seringue-aiguille selon la revendication 30, dans lequel ledit moyen de verrouillage (174) est mobile sur ledit rebord (117) pour capturer la portion (132) dudit porte-aiguille (114) qui s'étend dans ledit rebord.

32. Ensemble seringue-aiguille selon la revendication 7 ou 22, dans lequel ledit porte-aiguille (14, 114, 214, 314) comprend un bras latéral (32, 132, 232, 332) s'étendant latéralement à travers ladite cavité de plongeur (33, 133, 233) jusqu'audit corps (10, 110, 162, 210, 310) et ledit moyen de verrouillage est monté pour un mouvement dans et hors de ladite surface de guidage (60, 119, 160, 171, 219, 319) pour capturer et libérer ledit bras de porte-aiguille à l'extrémité distale de ladite surface de guidage.

33. Ensemble seringue-aiguille selon la revendication 32, dans lequel ledit moyen de verrouillage est monté en glissement dans un canal formé dans ledit corps et s'ouvrant dans ladite surface de guidage (119, 160, 219) à une extrémité du canal, et s'ouvrant via l'extrémité dudit corps (110, 162, 210) à l'autre extrémité du canal.

34. Ensemble seringue-aiguille selon la revendication 7 ou 22, dans lequel ledit plongeur (11, 111, 211, 311) comprend un moyen de contact s'étendant transversalement à travers l'intérieur dudit corps (10, 110, 162, 210, 310) et glissant le long de la surface intérieure dudit corps qui forme ladite surface de guidage (60, 119, 160, 219, 319) de manière à garder la configuration souhaitée de ladite surface et donc de garantir l'engagement du porte-aiguille (14, 114, 214, 314) avec ladite surface de guidage.

35. Ensemble seringue-aiguille selon la revendication 34, dans lequel ledit moyen de contact comprend une plaque circulaire (25, 125, 225, 325) formée d'un seul tenant avec ledit plongeur (11, 111, 211, 311).

36. Ensemble seringue-aiguille selon la revendication 35, dans lequel ledit moyen de contact comprend également une pluralité d'éléments longitudinaux formés d'un seul tenant avec ledit plongeur (11, 111, 311) et glissant le long de la surface intérieure dudit corps (10, 110, 162, 310) pour garder la configuration souhaitée de ladite surface.

37. Ensemble seringue-aiguille selon la revendication 7 ou 22, dans lequel ladite surface de guidage (60, 119, 160, 171, 219, 319) est formée dans la paroi dudit corps (10, 110, 162, 210, 310) et s'étend radialement à travers la paroi dudit corps.

38. Ensemble seringue-aiguille selon la revendication 37, qui comprend un manchon externe (216) télescopé sur ledit corps (210) et couvrant au moins une portion d'extrémité distale de ladite surface de guidage (219).

39. Ensemble seringue-aiguille selon la revendication 22, dans lequel ladite surface de guidage (60) comprend un moyen à son extrémité proximale pour résister au mouvement d'avancée dudit porte-aiguille (14, 114, 214) une fois qu'il est complètement rétracté.

40. Ensemble seringue-aiguille selon la revendication 22, dans lequel un cliquet (66, 119a, 219a) est situé à l'extrémité distale de ladite surface de guidage (60, 119, 160, 219).

41. Ensemble seringue-aiguille selon la revendication 22, dans lequel ledit moyen de verrouillage comprend une plaque glissante (151, 253) qui forme une portion d'extrémité distale d'une paroi latérale de ladite surface de guidage (119, 160, 219) lorsque ladite plaque est dans une position rétractée (ouverte), et qui bloque ladite surface de guidage et capture ledit porte-aiguille (114, 214) à l'extrémité distale de ladite surface de guidage lorsque ladite plaque est dans une position déployée (fermée).

42. Ensemble seringue-aiguille selon la revendication 7 ou 22, dans lequel ledit moyen de verrouillage comprend une tige de verrouillage longitudinale (167) montée en glissement dans la paroi dudit corps (162) pour se déplacer entre une position rétractée (ouverte) à l'extérieur de ladite surface de guidage (160) et une position déployée (fermée) dans laquelle la tige s'étend en travers dudit canal en spirale pour bloquer le canal et capturer ledit porte-aiguille (114) à l'extrémité distale de la surface de guidage.

43. Ensemble seringue-aiguille selon la revendication 42, dans lequel ladite surface de guidage est un canal en spirale (219), qui s'étend radialement à travers la paroi dudit corps (210) et qui comprend un manchon (216) télescopé sur ledit corps et couvrant ladite surface de guidage, les surfaces d'ajustement dudit corps et dudit manchon formant un passage longitudinal pour recevoir ladite tige de verrouillage.

44. Ensemble seringue-aiguille selon la revendication 7 ou 22, dans lequel ladite surface de guidage est une rainure en spirale et la portion (116) dudit corps (110) contenant ledit canal en spirale (119) a un diamètre intérieur plus grand que la portion distale dudit corps, et ledit moyen de verrouillage (150) glisse sur la surface externe de ladite portion distale du corps et sur la surface interne de la portion de corps contenant la rainure en spirale.

45. Ensemble seringue-aiguille selon la revendication 44, dans lequel la surface interne de la portion de corps (116) contenant la rainure en spirale (119) forme un canal longitudinal (153) pour recevoir et guider ledit moyen de verrouillage (150).

46. Ensemble seringue-aiguille selon la revendication 7 ou 32, dans lequel ledit moyen de verrouillage est formé d'un seul tenant avec ledit corps et comprend un bras et une tige de verrouillage pour un mouvement de va-et-vient entre une position de blocage au sein de ladite rainure en spirale et une position de non-blocage à l'extérieur de ladite rainure en spirale.
